# EUROPEAN PATENT SPECIFICATION

(11) **EP 3 482 495 B1**
(45) Date of publication and mention of the grant of the patent: **15.04.2020**
(21) Application number: 17745515.1
(22) Date of filing: 10.07.2017
(51) Int. Cl.: H03H 9/64, A61B 5/02

(54) **SURFACE ACOUSTIC WAVE RFID SENSOR FOR HEMODYNAMIC WEARABLES**
OBERFLÄCHENWELLEN-RFID-SENSOR FÜR HÄMODYNAMISCHE WEARABLES
CAPTEUR RFID À ONDES ACOUSTIQUES DE SURFACE POUR ACCESSOIRES VESTIMENTAIRES HÉMODYNAMIQUES

(30) Priority: 11.07.2016 US 201662360754 P
(43) Date of publication of application: 15.05.2019
(73) Proprietor: Epitronic Holdings Pte. Ltd., Singapore 079027 (SG)
(72) Inventor: RAM, Ayal, Singapore 079027 (SG)
(74) Representative: Lavoix
(86) International application number: PCT/IB2017/054143
(87) International publication number: WO 2018/011697

(56) References cited:
- WO-A1-2017/088560
- CN-A- 105 424 780
- US-A1- 2007 139 165
- US-A1- 2014 008 658
- PEARTON S J ET AL: "TOPICAL REVIEW; GaN-based diodes and transistors for chemical, gas, biological and pressure sensing; Topical Review", JOURNAL OF PHYSICS: CONDENSED MATTER, vol. 16, no. 29, 28 July 2004 (2004-07-28) , pages R961-R994, XP020059871, INSTITUTE OF PHYSICS PUBLISHING, BRISTOL, GB ISSN: 0953-8984, DOI: 10.1088/0953-8984/16/29/R02
- NAOTERU SHIGEKAWA ET AL: "Surface Acoustic Waves in Reverse-Biased AlGaN/GaN Heterostructures", IEEE TRANSACTIONS ON ELECTRON DEVICES, vol. 55, no. 7, 1 July 2008 (2008-07-01), pages 1585-1591, XP011216602, IEEE SERVICE CENTER, PISACATAWAY, NJ, US ISSN: 0018-9383
- LALINSKY T ET AL: "AlGaN/GaN based SAW-HEMT structures for chemical gas sensors", PROCEDIA ENGINEERING, vol. 5, 1 January 2010 (2010-01-01), pages 152-155, XP027483626, ELSEVIER, AMSTERDAM, NL ISSN: 1877-7058, DOI: 10.1016/J.PROENG.2010.09.402 [retrieved on 2010-10-25]
- FREDERIC MICHARD: "Hemodynamic monitoring in the era of digital health", ANNALS OF INTENSIVE CARE, vol. 6, 15, 2016, pages 1-7, XP002774730, DOI: 10.1186/s13613-016-0119-7 cited in the application

## Description

### TECHNICAL FIELD

In general, the present application relates to the field of electronic sensors based on surface acoustic wave (SAW) transducers and their use in detection and continuous monitoring of electrical fields generated by a human. In particular, the present application relates to the GaN/AlGaN zero-power SAW RFID sensor and its use in hemodynamic wearable devices.

### BACKGROUND

Personalised mobile medicine has been continuously advancing during the last few years due to the development of wearable sensors, which are capable of wirelessly providing essential medical information while remaining unobtrusive, comfortable, low cost, and easy to operate and interpret. Digital innovations are changing medicine, and hemodynamic monitoring is not an exception. In the nearest future, we can envision a world where clinicians will monitor patients with hemodynamic wearables or implantable sensors able to communicate with diagnostic clouds and integrate the historical, clinical, biological and physiologic information used in diagnostics and in prediction of adverse events, choosing the most rationale therapy and ensuring that it is delivered properly. The present application demonstrates that some of these ideas and products including personal gadgets and wearables for hemodynamic monitoring become a reality.

Frederic Michard ("Hemodynamic monitoring in the era of digital health", Annals of Intensive Care", 2016, 6:15) reviewed the modern techniques and devices used in hemodynamic monitoring. Most of these techniques and devices are based on pulse contour algorithms, which allow, for example, the computation of stroke volume and cardiac output from an arterial blood pressure curve. Their reliability mainly depends on the signal-to-noise ratio (quality) of the pressure signal and on changes in a vascular tone. In this context, DeBacker et al ("Arterial pressure-based cardiac output monitoring: a multicentre validation of the third-generation software in septic patients", Intensive Care Medicine 2011, 37:233-40) and Slagt et al ("Systematic review of uncalibrated arterial pressure waveform analysis to determine cardiac output and stroke volume variation", British Journal of Anaesthesia, 2014, 112:626-37) questioned accuracy and precision of these techniques and devices using thermodilution or echocardiography as reference methods, respectively.

The arterial pressure can be either invasively recorded from an arterial line, or noninvasively, from finger arteries, from the radial artery and from a brachial cuff. The arterial pressure waveform can also be captured from any bedside monitor by a cell phone camera, and hemodynamic parameters can be computed by a downloadable application, such as Captesia™ by Olivier Desebbe (available for Android). This app has not yet been approved for clinical use, but it illustrates the ability of digital technologies to automatically calculates the pulse-pressure variation (PPV) using a digital photograph of the arterial waveform from the monitor and selecting peaks and troughs of the arterial curve, thereby pushing the envelope of hemodynamic monitoring practices.

Microelectronic and nanoelectronic mechanical systems (MEMS and NEMS), are miniaturised pressure sensors based on piezoelectricity and piezoresistivity which are about to revolutionize the world. They are able to sense hemodynamic parameters with the high accuracy and high signal-to-noise ratio. Because they are relatively cheap in manufacture, tiny, non-invasive and wireless, such sensors may make continuous hemodynamic monitoring a reality beyond the operating room and intensive care units. These sensors may be used by many, from those at risk of hemodynamic deterioration to outpatients with chronic hypertension.

Moreover, the possibility of continuous ECG detection in the daily life is extremely important and invaluable for predictive health care in the modern society, where the heart distress signals could be detected as early as possible. Pulse-watches measuring the heart rate from a single point on a wrist using photoplethysmography (pulse oximetry) have been recently commercialised. Tom-Tom® Runner Cardio and Mio® Alpha watches use photoplethysmography to continuously measure the hemodynamic blood waves and heart rate. Their principle of operation is based on pulse transit time method with additional ECG-signal for calculation of blood pressure.

In general, an ECG is by definition a differential measurement. There is a gradient electric field distribution along the arm according to a bioelectric volume conductor body nature and to a dynamic electric field volume source representing the heart dipole function. This heart-dipole electric field creates a dynamic body surface potential map on the skin, which is measured differentially at least at two skin points representing the ECG signal. The distribution of the electrical field is computed with numerical methods outgoing from cardiac sources. The differential ECG signal is measured using the standard Ag/AgCl gel ECG electrodes when the reference electrode is placed on a patient's chest near his heart and the second electrode is moving gradually to the direction of fingertips between different points along the way from the heart to the fingertips. Normally, the differential signal reaches a maximum value at elbow and remains constant till fingertips. This experimental observation originates from the nature of the electric field distribution within a body. Since, the characteristic ECG peak (Q-toR peak) amplitude doesn't change below the elbow and in the wrist area, the signal distribution eminently hinders the actual ECG detection with any wearable elbow and wrist devices.

Therefore, there is a demand for a new type of wearable sensors, which would be based on a different detection principle. There are only two existing non-differential sensing techniques which are recently developed for cardiovascular monitoring on a chest or even on lower limbs. Nakayama *et al* (2011) and An *et al* (2012) described a microelectronic CMOS-based magnetic field sensors based on giant magneto impedance. These types of microelectronic devices were able to detect the magnetocardiography-single point signals (MCG) from chest but failed to detect the MCG signal from wrist.

Kado *et al* (2010) described optoelectronic detectors changing their optical properties, such as reflectance, as a function of e-field magnitude. Such transducers have already found their application in personal RFID systems, for example RedTacton® NTT. However, the detection of a lower limb ECG signals based on a non-differential skin potential or remote cardiovascular monitoring still remains unproven and challenging.

Pearton *et al* (2004) reviewed GaN-based diodes and transistors for chemical, gas, biological and pressure sensing, and demonstrated that AlGaN/GaN high-electron-mobility transistors (HEMTs) exhibit a very strong dependence of source/drain current in piezoelectric, polarization-induced, two-dimensional electron gas (2DEG) channels on variations in electrostatic boundary conditions of the free surface above the 2DEG (polar liquids, decomposition of hydrogen by catalytic Pt contacts and applied strain).

Shigekawa *et al* (2008) studied the properties and propagation of the surface-acoustic waves (SAWs) in reverse-biased AlGaN/GaN heterostructures grown on (0001) sapphire substrates by investigating the characteristics of SAW filters composed of interdigitated transducers (IDTs) made of interdigital Schottky and ohmic contacts.

CN 105424780 A disclosed a GaN-based HEMT sensor with a surface-functionalised membrane for detecting an analyte which is in contact with the surface of the sensor and measuring the analyte concentration. The thickness of the top layer is flexibly set in order to improve the detection range of the sensor.

### SUMMARY

The present invention is defined by a surface acoustic wave (SAW) radio-frequency identification (RFID) sensor chip based on a combination of a two-dimensional electron gas (2DEG) or two-dimensional hole gas (2DHG) conducting structure and surface acoustic wave (SAW) transducer according to claim 1, and a corresponding method according to claim 14. Embodiments of the invention are defined by the dependent claims. According to the invention the sensor contains a piezoelectric substrate, on which a multilayer heterojunction structure is deposited. This heterojunction structure comprises at least two layers, a buffer layer and a barrier layer, wherein both layers are grown from III-V single-crystalline or polycrystalline semiconductor materials. Interdigitated transducers (IDTs) transducing surface acoustic waves are installed on top of the barrier layer.

A conducting channel comprising a two-dimensional electron gas (2DEG) or a two-dimensional hole gas (2DHG) is formed at the interface between the buffer and barrier layers and may provide electron or hole current in the system between source and drain electrodes. In a particular embodiment, the heterojunction structure may be a three-layer structure consisting of two buffer layers and one barrier layer squeezed between said buffer layers like in a sandwich. This may lead to formation of the two-dimensional hole gas (2DHG) in the top buffer layer above the barrier layer which results in reversing polarity of the structure.

The capacitively-coupled (non-ohmic) source and drain contacts are connected to the formed 2DEG/2DHG channel and to electrical metallizations, wherein said metallizations may be placed on top of the structure and connect it to an electric circuit of the sensor. An optional dielectric layer may be deposited on top of the heterojunction structure. The open gate area of the 2DEG/2DHG structure is formed between the source and drain areas as a result of recessing or growing of the top layer to a specific thickness.

Since the source and drain contacts are non-ohmic (i.e. capacitively-coupled), the DC readout cannot be carried out. To electrically contact the 2DEG/2DHG channel underneath, about 5-20 nm bellow the metallizations, the AC-frequency regime must be used. In other words, the AC readout or impedance measurements of the electric current flowing through the 2DEG/2DHG-channel should be performed in this particular case. The capacitive coupling of the non-ohmic metal contacts with the 2DEG/2DHG channel is normally induced at the frequency higher than 30 kHz.

According to the invention, the multilayer heterojunction substrate of the present application is grown from a III-V single-crystalline or polycrystalline semiconductor materials, in particular GaN/AlGaN. In the specific case of the substrate grown from GaN/AlGaN, it has been experimentally and surprisingly found that the highest sensitivity of the sensor is achieved when thickness of the top recessed layer (GaN buffer layer or AlGaN barrier layer) in the open gate area between the source and drain contacts is 5-9 nm, preferably 6-7 nm, more preferably 6.2-6.4 nm. This recessed layer thickness corresponds to the pseudo-conducting current range between normally-on and normally-off operation mode of the 2DEG/2DHG conducting channel. In addition, surface roughness of the top recessed layer within the open gate area between the source and drain contacts has a roughness of about 0.2 nm or less, preferably 0.1 nm or less, more preferably 0.05 nm.

Further, in some embodiments, the present application provides the zero-power SAW RFID sensor, which is based on the GaN/AlGaN heterostructure, and its use in hemodynamic wearable devices. In an example, the sensor is a zero-power sensor remotely powered with the RF-energy and RFID-coded via the orthogonal frequency coding (OFC) method.

Various embodiments may allow various benefits, and may be used in conjunction with various applications. The details of one or more embodiments are set forth in the accompanying figures and the description below. Other features, objects and advantages of the described techniques will be apparent from the description and drawings and from the claims.

### BRIEF DESCRIPTION OF THE DRAWINGS

Disclosed embodiments will be understood and appreciated more fully from the following detailed description taken in conjunction with the appended figures. The drawings included and described herein are schematic and are not limiting the scope of the disclosure. It is also noted that in the drawings, the size of some elements may be exaggerated and, therefore, not drawn to scale for illustrative purposes. The dimensions and the relative dimensions do not necessarily correspond to actual reductions to practice of the disclosure.
**Fig. 1** schematically shows the quantum well at three different biasing conditions:
   **Fig. 1a****:** positive gate potential (+V_{G}) is much higher than threshold voltage (V_{T}),
   **Fig. 1b****:** 0V gate potential, and
   **Fig. 1c****:** negative gate potential (-V_{G}) is below threshold voltage (V_{T}).
**Fig. 2** schematically shows the dependence of the source-drain current (a charge carrier density) induced inside the 2DEG channel of a GaN/AlGaN HEMT on the thickness of the AlGaN barrier layer recessed in the open gate area.
**Fig. 3** illustrates a theory behind the 2DEG formation (charge neutrality combined with the lowest energy level) at the conduction band discontinuity.
**Fig. 4a** shows sensitivity of the PC-HEMT to ionic fluid for the 22-nm AlGaN barrier layer, normally grown and then recessed to 6-7 nm.
**Fig. 4b** shows sensitivity of the PC-HEMT to ionic fluid for the ultrathin AlGaN barrier layer grown to 6-7 nm, then recessed down to 5-6 nm and etched with plasma.
**Fig. 5a** schematically shows the formation of the 2DEG and 2DHG conducting channels in the Ga-face three-layer AlGaN/GaN PC-HEMT structure.
**Fig. 5b** schematically shows the formation of the 2DEG and 2DHG conducting channels in the N-face three-layer AlGaN/GaN PC-HEMT structure.
**Fig. 6** schematically shows the formation of the 2DEG conducting channel in the N-face three-layer AlGaN/GaN PC-HEMT structure with an ultrathin Al(GaN)N layer for improved confinement.
**Fig. 7a** schematically shows the input interdigitated transducer (IDT)-based SAW device.
**Fig. 7b** schematically shows the IDT and its characteristic parameters: length (L), width (W) and acoustic wavelength (λ).
**Fig. 7c** shows the bandwidth (B) of the SAW as a function of the number of the IDTs and the frequency (f), where f₀ is the centre frequency.
**Fig. 8** schematically shows a SAW RFID sensor of an embodiment with 2DEG IDTs on a GaN/AlGaN heterostructure.
**Fig. 9** schematically shows the basic topology of the sensor of an embodiment, wherein bands (100) and (104) in are assigned to metal structures, such as metal IDTs and metal electrodes, respectively, bands (103) represent 2DEG structures, band (102) shows the PC-HEMT-like structure and white (background) area (101) stands for the AlGaN/GaN-substrate.
**Fig. 10** schematically shows another basic topographic 2DEG-SAW sensor configuration of an embodiment. On the left, the PC-HEMT-like structure contains a major pseudo-conducting 2DEG part (102) and non-recessed 2DEG part (103). Ohmic contacts are represented by bands (105).
**Fig. 11** schematically shows still another configuration of the 2DEG-SAW sensor of an embodiment. In this configuration, the meander antenna parts for the signal and ground charge are separated by counter shortcutting each other via the parasitic 2DEG lines at the top and at the bottom of the shown layout.
**Fig. 12** schematically shows yet further configuration of the 2DEG-SAW sensor. In this simplest configuration, the pseudo-conducting 2DEG area (102) is covering all the emitter-receiver IDTs area and is optionally connected via the ohmic contacts (105) to the gate electrode (104), which is in turn connected to the IDTs (100).
**Fig. 13** schematically shows a zero-power SAW RFID sensor of an embodiment with a remote readout.
**Fig. 14** schematically shows an optoelectronic sensor of an embodiment for remote readout.

### DETAILED DESCRIPTION

In the following description, various aspects of the present application will be described. For purposes of explanation, specific configurations and details are set forth in order to provide a thorough understanding of the present application. However, it will also be apparent to one skilled in the art that the present application may be practiced without the specific details presented herein. Furthermore, well-known features may be omitted or simplified in order not to obscure the present application.

The term "comprising", used in the claims, is "open ended" and means the elements recited, or their equivalent in structure or function, plus any other element or elements which are not recited. It should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising x and z" should not be limited to devices consisting only of components x and z. Also, the scope of the expression "a method comprising the steps x and z" should not be limited to methods consisting only of these steps.

Unless specifically stated, as used herein, the term "about" is understood as within a range of normal tolerance in the art, for example within two standard deviations of the mean. In one embodiment, the term "about" means within 10% of the reported numerical value of the number with which it is being used, preferably within 5% of the reported numerical value. For example, the term "about" can be immediately understood as within 10%, 9%, 8%, 7%, 6%, 5%, 4%, 3%, 2%, 1%, 0.5%, 0.1%, 0.05%, or 0.01% of the stated value. In other embodiments, the term "about" can mean a higher tolerance of variation depending on for instance the experimental technique used. Said variations of a specified value are understood by the skilled person and are within the context of the present invention. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges, for example from 1-3, from 2-4, and from 3-5, as well as 1, 2, 3, 4, 5, or 6, individually. This same principle applies to ranges reciting only one numerical value as a minimum or a maximum. Unless otherwise clear from context, all numerical values provided herein are modified by the term "about". Other similar terms, such as "substantially", "generally", "up to" and the like are to be construed as modifying a term or value such that it is not an absolute. Such terms will be defined by the circumstances and the terms that they modify as those terms are understood by those of skilled in the art. This includes, at very least, the degree of expected experimental error, technical error and instrumental error for a given experiment, technique or an instrument used to measure a value.

As used herein, the term "and/or" includes any and all combinations of one or more of the associated listed items. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

It will be understood that when an element is referred to as being "on", "attached to", "connected to", "coupled with", "contacting", etc., another element, it can be directly on, attached to, connected to, coupled with or contacting the other element or intervening elements may also be present. In contrast, when an element is referred to as being, for example, "directly on", "directly attached to", "directly connected to", "directly coupled" with or "directly contacting" another element, there are no intervening elements present. It will also be appreciated by those of skill in the art that references to a structure or feature that is disposed "adjacent" another feature may have portions that overlap or underlie the adjacent feature.

The polarization doped high-electron-mobility transistor (HEMT) is a field effect transistor (FET) in which two layers of different bandgap and polarisation field are grown upon each other forming the heterojunction structure. In one aspect, the sensor of the present application contains a piezoelectric substrate comprising the HEMT-like multilayer heterojunction structure. This structure is essentially based on at least two layers of III-V semiconductor materials, such as gallium nitride (GaN) and aluminium gallium nitride (AlGaN). As a consequence of the discontinuity in the polarisation field, surface charges are created at the interface between the layers of the heterojunction structure. If the induced surface charge is positive, electrons will tend to compensate the induced charge resulting in the formation of the channel. Since the channel electrons are confined in a quantum well in an infinitely narrow spatial region at the interface between the layers, these electrons are referred to as a two-dimensional electron gas (2DEG). This special confinement of the channel electrons in the quantum well actually grants them two-dimensional features, which strongly enhance their mobility surpassing the bulk mobility of the material in which the electrons are flowing.

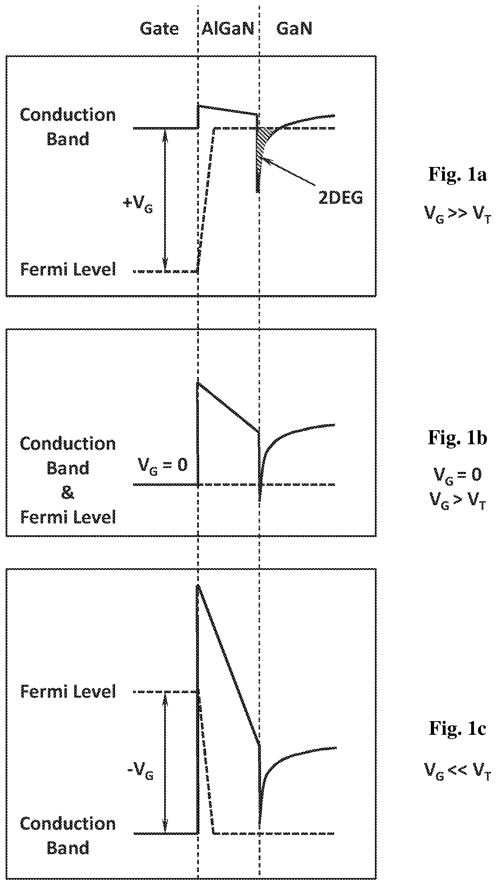

**Figs. 1a-1c** schematically shows the quantum well at three different biasing conditions starting from the positive gate potential (V_{G}), much higher than the threshold voltage (V_{T}), and going down to the 0V gate potential and further to the negative values below the threshold voltage. The V_{T} is defined as a voltage, which is required to populate electrons at the interface between the GaN layer and the AlGaN layers, thereby creating conductivity of the 2DEG channel. Since the 2DEG channel electrons occupy energy levels below the Fermi level, the Fermi level in a quantum well is located above several energy levels when V_{G} >> V_{T} (**Fig**. **1a**)**.** This enables high population of channel electrons and consequently, high conductivity. The 2DEG channel is turned on in this case. However, when V_{G} decreases to 0V (**Fig**. **1b**), the Fermi level also drops with respect to the quantum well. As a result, much fewer electron energy levels are populated and the amount of the 2DEG channel electrons significantly decreases. When V_{G} much less than V_{T} (**Fig**. **1c**)**,** all electron energy levels are above the Fermi level, and there is no the 2DEG electrons below the gate. This situation is called "channel depletion", and the channel is turned off.

Many commercially available HEMTs based on the layers of III-V semiconductor materials have a negative value of V_{T}, resulting in a "normally-on" operation mode at 0V gate potential. They are called "depletion-mode" semiconductor transistors and used in various power switching applications when the negative voltage must be applied on the gate in order to block the current. However, for safe operation at high voltage or high power density, in order to reduce the circuit complexity and eliminate standby power consumption, the transistors with "normally-off" characteristics are preferred. The high voltages and high switching speeds allow smaller, more efficient devices, such as home appliances, communications and automobiles to be manufactured. To control the density of electrons in the 2DEG channel and to switch the HEMT on and off, the voltage at the gate of the transistor is normally regulated.

Several techniques to manufacture the normally-off semiconductor structures have been reported. Burnham *et al* (2010) proposed normally-off structures of the recessed gate type. In this structure, the AlGaN barrier layer is etched and the gate is brought closer to the interface between the AlGaN barrier layer and the GaN buffer layer. As the gate approaches the interface between the layers, the V_{T} increases. Thus, the normally-off operation of the 2DEG conducting channel is achieved once the depletion region reaches the interface and depletes the 2DEG channel at zero gate voltage. The major advantages of these structures are relatively lower power consumption, lower noise and simpler drive circuits. They are currently used, for example, in microwave and millimetre wave communications, imaging and radars.

Chang *et al* (2009) proposed instead of etching the relatively thick barrier layer to approach the AlGaN/GaN interface, to use a very thin AlGaN barrier. This structure also achieves the normally-off operation of the 2DEG channel by approaching the gate towards the AlGaN/GaN interface. Chen *et al* (2010) proposed to use the fluorine-based plasma treatment method. Although many publications have adopted various methods to achieve normally-off devices with minimum impact on the drain current, they unfortunately sacrificed device turn-on performance.

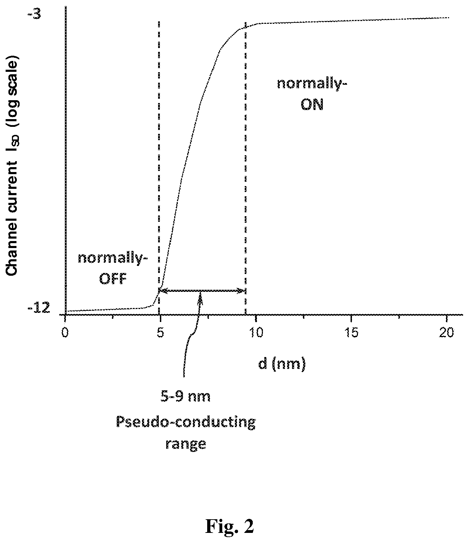

**Fig. 2** shows the dependence of the source-drain current (a charge carrier density) on the barrier layer thickness recessed in the open gate area. As seen from the figure, structures that have a thickness of the barrier layer in the open gate area larger than 9 nm form normally-on 2DEG channels. In such structures, due to the inherent polarisation effects present in the III-V materials, a thin sheet of charges is induced at the top and bottom of the interfaces of the barrier layer. As a result, a high electric field is induced in the barrier layer, and surface donor states at the top interface start donating electrons to form the 2DEG channel at the proximity of the heterojunction interface without the application of a gate bias. These structures therefore act as normally-on devices. On the other hand, the structures that have a thickness of the barrier layer in the open gate area lower than about 5 nm act as normally-off devices.

The present application describes embodiments of a microelectronic sensor or sensor chip based on a combination of a two-dimensional electron gas (2DEG) or two-dimensional hole gas (2DHG) structure and surface acoustic wave (SAW) transducer. In some embodiments, the sensor may contain a piezoelectric substrate, on which the multilayer heterojunction structure may be deposited. This heterojunction structure comprises at least two layers, a buffer layer and a barrier layer, wherein both layers are grown from the aforementioned III-V single-crystalline or polycrystalline semiconductor materials. Interdigitated transducers (IDTs) transducing surface acoustic waves may be installed on top of the barrier layer. The multilayer heterojunction structure of the present application is grown from any available III-V single-crystalline or polycrystalline semiconductor materials, such as GaN/AlGaN. In the specific case of the substrate grown from GaN/AlGaN, it has been experimentally found that the highest sensitivity of the sensor is achieved when thickness of the top recessed layer (GaN buffer layer or AlGaN barrier layer) in the open gate area between the source and drain contacts is 5-9 nm, preferably 6-7 nm, more preferably 6.2-6.4 nm. In addition, it was also found that the sensor exhibits its highest sensitivity when surface roughness of the top recessed layer is about 0.2 nm or less, preferably 0.1 nm or less, more preferably 0.05 nm.

Thus, the top layer recessed or grown in the open gate area to 5-9 nm must be optimised for significantly enhancing sensitivity of the sensor. This specific thickness of the barrier layer was surprisingly found to correspond to the "pseudo-conducting" current range between normally-on and normally-off operation modes of the 2DEG channel and requires further explanation.

"Pseudo-conducting" (to distinguish from normally-conducting) current range of the 2DEG channel is defined as an operation range of the channel between its normally-on and normally-off operation modes. "Trap states" are states in the band-gap of a semiconductor which trap a carrier until it recombines. "Surface states" are states caused by surface reconstruction of the local crystal due to surface tension caused by some crystal defects, dislocations, or the presence of impurities. Such surface reconstruction often creates "surface trap states" corresponding to a surface recombination velocity. Classification of the surface trap states depends on the relative position of their energy level inside the band gap. The surface trap states with energy above the Fermi level are acceptor-like, attaining negative charge when occupied. However, the surface trap states with energy below the Fermi level are donor-like, positively charged when empty and neutral when occupied. These donor-like surface trap states are considered to be the source of electrons in the formation of the 2DEG channel. They may possess a wide distribution of ionization energies within the band gap and are caused by redox reactions, dangling bonds and vacancies in the surface layer. A balance always exists between the 2DEG channel density and the number of ionised surface donors which is governed by charge neutrality and continuity of the electric field at the interfaces.

Thus, the donor-like surface traps at the surface of the barrier layer are one of the most important sources of the 2DEG in the channel. However, this only applies for a specific barrier layer thickness. In a relatively thin barrier layer, the surface trap state is below the Fermi level. However, as the barrier layer thickness increases, the energy of the surface trap state approaches the Fermi energy until it coincides with it. The thickness of the barrier layer corresponding to such situation is defined as "critical". At this point, electrons filling the surface trap state are pulled to the channel by the strong polarisation-induced electric field found in the barrier to form the 2DEG instantly.

If the surface trap states are completely depleted, further increase in the barrier layer thickness will not increase the 2DEG density. Actually, if the 2DEG channel layer fails to stretch the barrier layer, the later will simply relax. Upon relaxation of the barrier layer, many crystal defects are created at the interface between the buffer and barrier layers, and the piezoelectric polarisation instantly disappears causing deterioration in the 2DEG density.

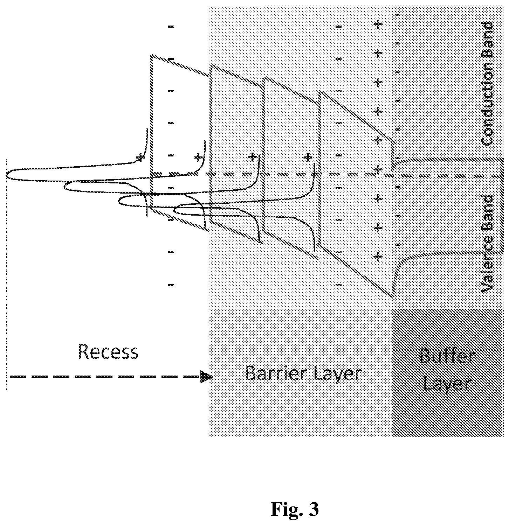

In order to illustrate the above phenomenon of the pseudo-conducting current, reference is now made to **Figs. 2** and **3****.** As described above, **Fig. 2** shows the dependence of the source-drain current (a charge carrier density) on the recessed AlGaN barrier layer thickness. Energy equilibrium between the donor surface trap states and AlGaN tunnel barrier leads to the 2DEG formation (charge neutrality combined with the lowest energy level) at the conduction band discontinuity. As explained above, decrease in the thickness of the barrier layer results in increase of the energy barrier. As a result, the ionisable donor-like surface trap states, which are responsible for electron tunnelling from the surface to 2DEG, drift bellow the Fermi level, thereby minimizing the electron supply to the 2DEG channel. This theoretical situation is further illustrated in **Fig. 3****.** Therefore, the recess of the AlGaN layer from 9 nm to 5 nm leads to huge drop in conductivity of the two-dimensional electron gas for six orders of magnitude.

Thus, the mechanism of the 2DEG depletion based on recessing the barrier layer is strongly dependent on the donor-like surface trap states (or total surface charge). As the thickness of the barrier layer decreases, less additional external charge is needed to apply to the barrier layer surface in order to deplete the 2DEG channel. There is a critical (smallest) barrier thickness, when the 2DEG channel is mostly depleted but still highly conductive due to a combination of the energy barrier and the donor surface trap states energy. At this critical thickness, even the smallest energy shift at the surface via any external influence, for example an acoustic wave propagating along the surface, leads immediately to the very strong 2DEG depletion. As a result, the surface of the barrier layer at this critical thickness is extremely sensitive to any smallest change in the electrical field of the surroundings.

Thus, recess of the barrier layer from 9 nm down to 5 nm significantly reduced the 2DEG density, brought the sensor to the "near threshold" operation and resulted in highly increased surface charge sensitivity. The specific 5-9 nm thickness of the barrier layer responsible for the pseudo-conducting behaviour of the 2DEG channel gives the sensor an incredible sensitivity.

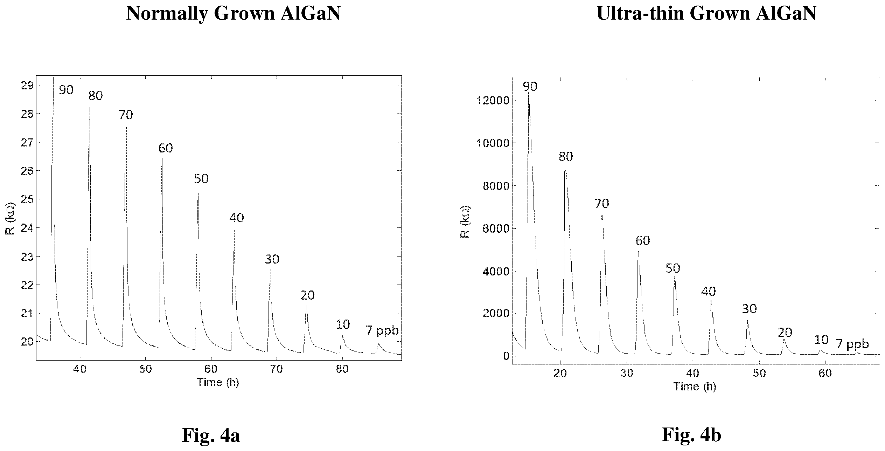

For example, the heterojunction structure with a 22-nm grown AlGaN layer, subjected to short plasma activation (60 s) and recessed to 6-7 nm, is compared with the ultrathin grown 6-7 nm heterojunction structure having the AlGaN barrier layer recessed to 5-6 nm and etched with plasma for 450 s. In the first case, the AlGaN barrier layer is initially not recessed, but instead the 2-3-nm SiN layer (known as a "GaN cap layer") is cracked and the surface states are ionised. The AlGaN barrier layer in the second case is recessed down to 6.3 nm and etched with plasma for 450 s. As shown in **Figs. 4a** and **4b****,** the difference in sensitivity between the two structures was found to be almost 10³ times in the favour of the recessed structure.

In addition to the recessed or grown top barrier layer thickness, roughness of the barrier layer surface is another very important parameter that has not been previously disclosed. It has been surprisingly found that that the roughness of the top AlGaN barrier layer surface (in the open gate sensitive area) bellow 0.2 nm prevents scattering of the donor-like surface trap states. Thus, combination of these two features: 5-9 nm thickness of the top AlGaN barrier layer in the open gate area and strongly reduced roughness of its surface make the sensor incredibly sensitive.

In a further aspect, the hetero-junction structure may be a three-layer structure consisting of two buffer layers and one barrier layer squeezed between said buffer layers like in a sandwich, wherein the top layer is a buffer layer. This may lead to formation of the two-dimensional hole gas (2DHG) in the top buffer layer above the barrier layer which results in reversing polarity of the transistor compared to the two-layer structure discussed above.

In general, polarity of III-V nitride semiconductor materials strongly affects the performance of the transistors based on these semiconductors. The quality of the wurtzite GaN materials can be varied by their polarity, because both the incorporation of impurities and the formation of defects are related to the growth mechanism, which in turn depends on surface polarity. The occurrence of the 2DEG/2DHG and the optical properties of the hetero-junction structures of nitride-based materials are influenced by the internal field effects caused by spontaneous and piezo-electric polarizations. Devices in all of the III-V nitride materials are fabricated on polar {0001} surfaces. Consequently, their characteristics depend on whether the GaN layers exhibit Ga-face positive polarity or N-face negative polarity. In other words, as a result of the wurtzite GaN materials polarity, any GaN layer has two surfaces with different polarities, a Ga-polar surface and an N-polar surface. A Ga-polar surface is defined herein as a surface terminating on a layer of Ga atoms, each of which has one unoccupied bond normal to the surface. Each surface Ga atom is bonded to three N atoms in the direction away from the surface. In contrast, an N-polar surface is defined as a surface terminating on a layer of N atoms, each of which has one unoccupied bond normal to the surface. Each surface N atom is also bonded to three Ga atoms in the direction away from the surface. Thus, the N-face polarity structures have the reverse polarity to the Ga-face polarity structures.

As described above for the two-layer heterojunction structure, the barrier layer is always placed on top of the buffer layer. The layer which is therefore recessed is the barrier layer, specifically the AlGaN layer. As a result, since the 2DEG is used as the conducting channel and this conducting channel is located slightly below the barrier layer (in a thicker region of the GaN buffer layer), the hetero-junction structure is grown along the {0001}-direction or, in other words, with the Ga-face polarity. However, as explained above, the physical mechanism that leads to the formation of the 2DEG is a polarisation discontinuity at the AlGaN/GaN interface, reflected by the formation of the polarisation-induced fixed interface charges that attract free carriers to form a two-dimensional carrier gas. It is a positive polarisation charge at the AlGaN/GaN interface that attracts electrons to form 2DEG in the GaN layer slightly below this interface.

As noted above, polarity of the interface charges depends on the crystal lattice orientation of the hetero-junction structure, i.e. Ga-face versus N-face polarity, and the position of the respective AlGaN/GaN interface in the hetero-junction structure (above or below the interface). Therefore, different types of the accumulated carriers can be present in the hetero-junction structure of the embodiments.

In case of the three-layer hetero-junction structure, there are four possible configurations:

### Ga-face polarity

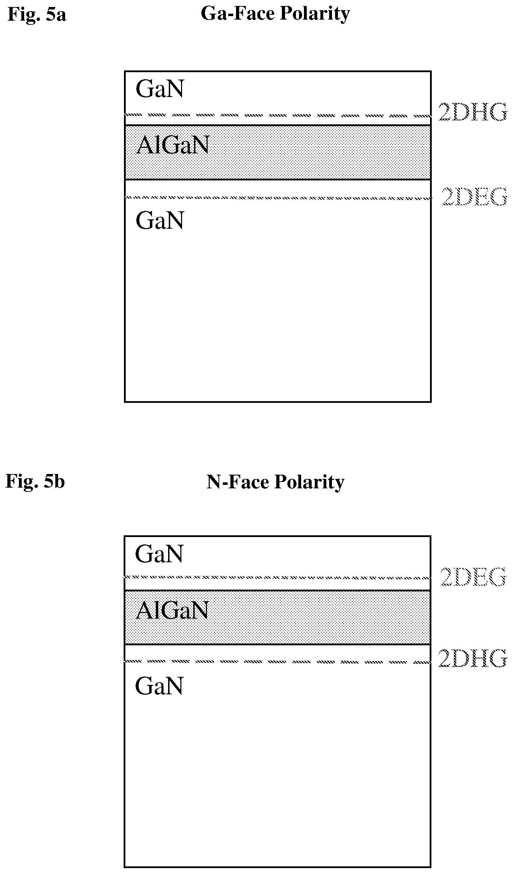

1) The Ga-face polarity is characterised by the 2DEG formation in the GaN layer below the AlGaN barrier layer. This is actually the same two-layer configuration as described above, but with addition of the top GaN layer. In this configuration, the AlGaN barrier layer and two GaN buffer layers must be nominally undoped or n-type doped.
2) In another Ga-face configuration shown in **Fig. 5a****,** in order to form the conducting channel comprising a two-dimensional hole gas (2DHG) in the top GaN layer above the AlGaN barrier layer in the configuration, the AlGaN barrier layer should be p-type doped (for example, with Mg or Be as an acceptor) and the GaN buffer layer should be also p-type doped with Mg, Be or intrinsic.

### N-face polarity

3) The N-face polarity is characterised by the 2DEG formation in the top GaN layer above the AlGaN barrier layer, as shown in **Fig. 5b****.** In this case, the AlGaN barrier layer and two GaN buffer layers must be nominally undoped or n-type doped.
4) The last configuration assumes that the 2DHG conducting channel is formed in the buffer GaN layer below the AlGaN barrier layer. The top GaN layer may be present (three-layer structure) or not (two-layer structure) in this case. The AlGaN barrier layer must be p-type doped (for example, with Mg or Be as an acceptor) and the bottom GaN layer should be also p-type doped with Mg, Be or intrinsic.

Thus, there are four hetero-junction three-layer structures implemented in the transistor of the embodiments, based on the above configurations:
A. **Ga-Face** GaN/AlGaN/GaN heterostructure with the 2DEG formed in the GaN buffer layer below the AlGaN barrier layer. In this case, the top GaN layer may be omitted to obtain the two-layer structure. For the three-layer structure, the top GaN layer must be recessed to 1-9 nm thickness in the open gate area or grown with this low thickness, with the roughness below 0.2 nm, and the thickness of the AlGaN barrier can be adjusted properly during growth.
B. **Ga-Face** GaN/AlGaN/GaN heterostructure with the 2DHG conducting channel formed in the top GaN layer above the AlGaN barrier layer. The top GaN layer must be recessed to 5-9 nm thickness in the open gate area with the roughness below 0.2 nm, and the thickness of the AlGaN barrier layer can be adjusted properly. P-type doping concentrations of the GaN layer and AlGaN barrier have to be adjusted; the 2DHG has to be contacted (in the ideal case by ohmic contacts).
C. **N-Face** GaN/AlGaN/GaN heterostructure with the 2DEG in the top GaN layer above the AlGaN barrier layer. The top GaN layer must be recessed to 5-9 nm thickness in the open gate area with the roughness below 0.2 nm. Thickness of the AlGaN barrier can be adjusted during growth. N-type doping levels of the GaN buffer layer and the AlGaN barrier layer must be adjusted; the 2DEG has to be contacted (in the ideal case by ohmic contacts).
D. **N-Face** GaN/AlGaN/GaN heterostructure with the 2DHG in the GaN buffer layer below the AlGaN barrier layer. In this case, the top GaN layer may be omitted to obtain the two-layer structure. In both, the two-layer and three-layer configurations, the top GaN layer must be recessed to 1-9 nm thickness in the open gate area with the roughness below 0.2 nm, and the thickness of the AlGaN barrier can be adjusted properly.

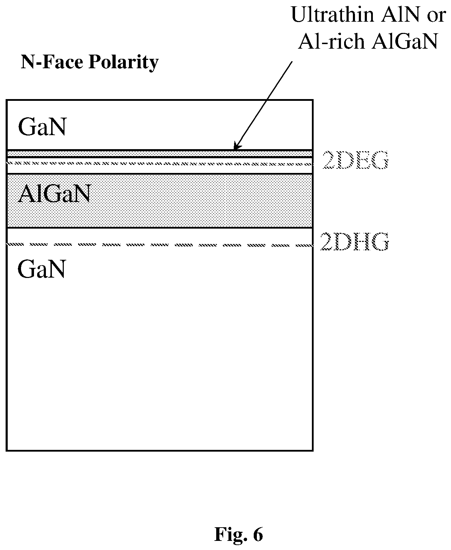

In all the above structures, the deposition of a dielectric layer on top might be beneficial or even necessary to obtain a better confinement (as in case of the N-face structures). As shown in **Fig. 6****,** for the above "C" structure, it may be even more beneficial to include an ultrathin (about 1 nm) AlN or AlGaN barrier layer with high Al-content on top of the 2DEG channel to improve the confinement.

The preferable structures of the embodiments are structures "B" and "C". In the structure "B", the 2DHG conducting channel formed in the top GaN layer, which has a higher chemical stability (particularly towards surface oxidation) than the AlGaN layer. Concerning the structure "C", the 2DEG conducting channel might be closer to the surface. Therefore, the electron mobility might be lower than in the 2DEG structure with the Ga-face polarity. In general, the polarity of the heterostructure can be adjusted by the choice of the substrate (e.g. C-face SiC) or by the growth conditions.

Another important feature of the sensor of the present application is that an electrical connection of the heterojunction structure to the 2DEG or 2DHG channel is realised via capacitive coupling to the electrical metallizations through a Schottky barrier contact. "Capacitive coupling" is defined as an energy transfer within the same electric circuit or between different electric circuits by means of displacement currents induced by existing electric fields between circuit/s nodes. In general, ohmic contacts are the contacts that follow Ohm's law, meaning that the current flowing through them is directly proportional to the voltage. Non-ohmic contacts however do not follow the same linear relationship of the Ohm's law. In other words, electric current passing through non-ohmic contacts is not linearly proportional to voltage. Instead, it gives a steep curve with an increasing gradient, since the resistance in that case increases as the electric current increases, resulting in increase of the voltage across non-ohmic contacts. This is because electrons carry more energy, and when they collide with atoms in the conducting channel, they transfer more energy creating new high-energy vibrational states, thereby increasing resistance and temperature.

When electrical metallizations are placed over single-crystalline or polycrystalline semiconductor material, the "Schottky contact" or "Schottky barrier contact" between the metal and the semiconductor occurs. Energy of this contact is covered by the Schottky-Mott rule, which predicts the energy barrier between a metal and a semiconductor to be proportional to the difference of the metal-vacuum work function and the semiconductor-vacuum electron affinity. However, this is an ideal theoretical behaviour, while in reality most interfaces between a metal and a semiconductor follow this rule only to some degree. The boundary of a semiconductor crystal abrupt by a metal creates new electron states within its band gap. These new electron states induced by a metal and their occupation push the centre of the band gap to the Fermi level. This phenomenon of shifting the centre of the band gap to the Fermi level as a result of a metal-semiconductor contact is defined as "Fermi level pinning", which differs from one semiconductor to another. If the Fermi level is energetically far from the band edge, the Schottky contact would preferably be formed. However, if the Fermi level is close to the band edge, an ohmic contact would preferably be formed. The Schottky barrier contact is a rectifying non-ohmic contact, which in reality is almost independent of the semiconductor or metal work functions.

Thus, a non-ohmic contact allows electric current to flow only in one direction with a non-linear current-voltage curve that looks like that of a diode. On the contrary, an ohmic contact allows electric current to flow in both directions roughly equally within normal device operation range, with an almost linear current-voltage relationship that comes close to that of a resistor (hence, "ohmic").

Since the source and drain contacts are non-ohmic (i.e. capacitively-coupled), the DC readout cannot be carried out. To electrically contact the 2DEG/2DHG channel underneath, about 5-20 nm bellow the metallizations, the AC-frequency regime must be used. In other words, the AC readout or impedance measurements of the electric current flowing through the 2DEG/2DHG-channel should be performed in this particular case. The capacitive coupling of the non-ohmic metal contacts with the 2DEG/2DHG channel becomes possible only if sufficiently high AC frequency, higher than 30 kHz, is applied to the metallizations. To sum up, the electrical metallizations, which are capacitively coupled to the 2DEG/2DHG channel utilise the known phenomenon of energy transfer by displacement currents. These displacement currents are induced by existing electrical fields between the electrical metallizations and the 2DEG/2DHG conducting channel operated in the AC frequency mode through the Schottky contact as explained above.

Surface acoustic wave (SAW) resonators are a class of MEMS based on the modulation of surface acoustic waves. The detection mechanism for SAW resonators utilizes changes in the amplitude, velocity, or phase of a SAW propagating along the substrate due to changes to the characteristics of the propagation path. In general, the energy of the SAW is normally concentrated in a surface region with a thickness of less than 1.5 times its wavelength. Therefore, the SAW resonator is extremely sensitive to its environment.

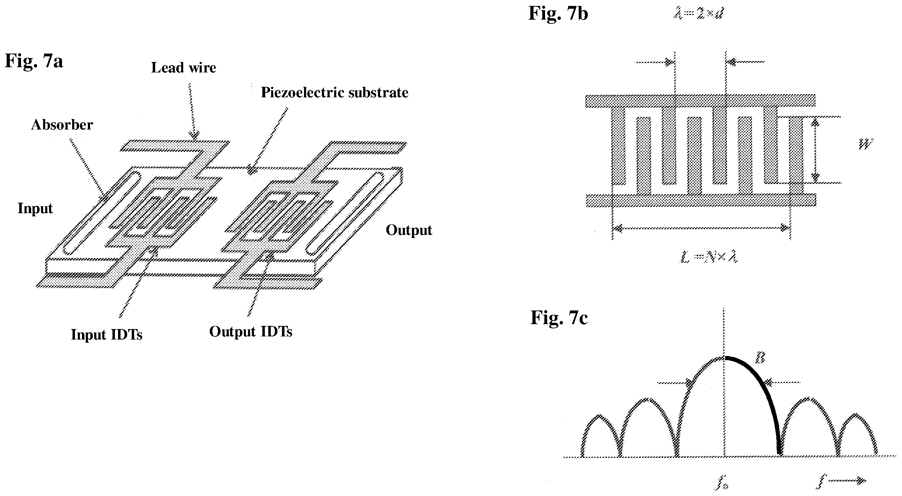

The principle of the inter-digitated transducer (IDT)-based SAW sensor is shown in **Figs. 7a-7c****.** A pair of IDTs, fabricated on the GaN/AlGaN substrate, serves as input and output ports of the signals. Fabrication of the SAW sensors comprises material selection, patterning, dicing, functionalisation and final packaging.

In general, the SAW sensors are designed by choosing the desired frequency and bandwidth of operation. The SAW can be expressed as a complex value *γ* = *α* + *iβ*, wherein the attenuation constant *α* and propagation constant *β* = *2π* / *λ* are important design parameters of the SAW sensor (*λ* is the acoustic wavelength). Another important design parameter is the electromechanical coupling coefficient *K*², which is a measure of the efficiency for converting an applying microwave signal into mechanical energy. These parameters will determine the magnitude of the observed changes in the SAW phase velocity and attenuation of the SAW intensity.

As shown in **Figs. 7b-7c****,** the operation frequency of the SAW sensor f₀ can be chosen by properly choosing the inter-digital finger spacing *d* such that *f₀* = *v*/*d*, where *v* is the wave propagation velocity in the specific substrate. Consequently, the dimensions of the designed SAW sensor depend on the chosen operating frequency, which can vary from a micrometre for 1-10 GHz to millimetres for kHz-MHz operation. SAW sensors operating in the GHz range can be readily designed and easily integrated with RF, the diverse MMIC, and the micro-strip circuits for low power wireless remote sensing. The bandwidth of the acoustic wave is given by *B* = *v* / *2Nd,* where *N* is number of inter-digital fingers, as shown in **Fig. 7b****.**

The aforementioned GaN/AlGaN-based systems are almost ideal materials for the SAW sensors due to their high SAW propagation velocity of about 4000 m/s, high electromechanical coupling coefficients, and their compatibility with the RF electronic integration. These materials also show excellent resistance to humidity and chemical etching. The GaN/AlGaN heterostructures described above exhibit a strong piezoelectric effect and have been used to fabricate the ultra-sensitive SAW-microbalances, exploiting the influence of mass accumulation on the SAW propagation. The high electromechanical coupling coefficients of the GaN/AlGaN substrate (*K²_{eff}* = 0.001-0.002), in combination with the low acoustic loss and SAW high velocity, enable their use in high-frequency and diverse low-loss RF applications. Therefore, the GaN/AlGaN-based SAW resonators and sensors operating up to the 10 GHz range can be designed and integrated with any wireless remote sensing applications.

Thus, using the GaN/AlGaN heterostructure as the piezoelectric substrate for the SAW sensors may result in a considerable improvement of the detection limit and in a high selectivity. This is a result of the 2DEG/2DHG's sensitivity to any proximal surface charge and a high mass sensitivity, as explained above. Thus, the GaN/AlGaN heterostructures and Schottky diodes can be integrated with a SAW sensor to create a rather unique resonant SAW tuning device with low acoustic loss, low loss RF performance and high frequency. The 2DEG/2DHG in a GaN/AlGaN structure and in a SAW propagation path interacts with the lateral electric field, resulting in ohmic loss, which attenuates and slows the SAW. This mechanism can be used to tune the SAW propagation velocity.

However, to combine the 2DEG/2DHG with the SAW achieving a maximal sensory effectiveness, some physical aspects must be taken into account. The actual functional combination of the 2DEG/2DHG with the SAW requires complete or partial removal, depletion or appropriate patterning of the 2DEG/2DHG in the quantum-well channel in the acoustic wave propagation region. The high charge conductivity in the conducting 2DEG/2DHG channel can screen the electric field and reduce the acoustoelectric transductions in the IDTs.

The metallic IDTs introduce inherent mass loading effects and triple-transit-interference (TTI), reducing the signal-to-noise ratio. In conventional SAW sensors, the average SAW propagation velocity under the metallic IDTs will be reduced from the free-surface value and will result in a reduction of its centre frequency with an increased amplitude and phase rippling across the bandpass due to signal reflection from the metallic IDTs.

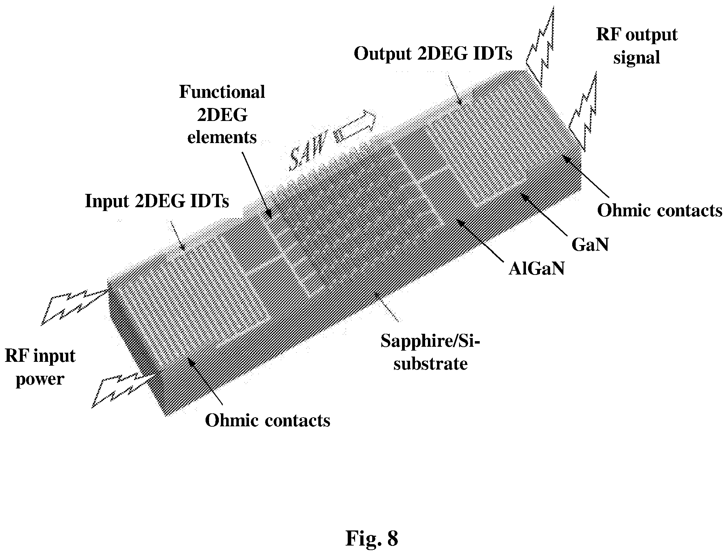

The aforementioned problems can be actually overcome by using the 2DEG-or 2DHG-based IDT fingers whilst also increasing the sensor sensitivity. The radio-frequency (RF) characteristics of the SAW device with planar 2DEG/2DHG IDTs are nearly equal to those using metallic IDTs with Schottky contact. Moreover, mass-loading effects and the TTI are suppressed when using the 2DEG/2DHG-based transducers instead of metallic IDTs. Also, the detection area of the SAW sensor or resonator can be right on top of the planar 2DEG/2DHG IDTs rather than in a separate SAW propagation area in between the IDTs. **Fig. 8** schematically shows a sensor with 2DEG/2DHG IDTs on a GaN/AlGaN heterostructure.

In general, when metallic IDTs are placed on microcrystalline semiconductor material, the Schottky contact is formed between the metal and the semiconductor, as explained above (regarding non-ohmic contacts). Considering the charge sensitivity mechanism in 2DEG/2DHG-based SAW, other charge sensitive 2DEG/2DHG areas can be added that operate in either the resonant centre frequency or in other resonant modes. These additional patterned 2DEG/2DHG areas will further enhance the resonant changes in the main SAW sensor through their charge gating. By studying the different signal shapes for different resonant modes, a selective sensing can be introduced. Besides the charge sensitive 2DEG/2DHG IDTs, other functional 2DEG/2DHG-elements, such as, for example, a 2DEG/2DHG-Schottky diode and a 2DEG/2DHG-planar non-symmetrical diode, nanowires and high electron mobility transistors can be placed between and connected with input and output IDTs operating in a resonant filter mode, as illustrated in the **Fig. 8****.** The electrical characteristics of such functional elements are modulated due to acoustoelectric transduction, which is time correlated (synchronized with IDTs). This results in a minimal electric loss and a specified signal shape for the SAW resonance. Through the electrostatic field gating, for example by redox processes occurring on the surface, this resonant SAW filter mode is easily affected (frequency, amplitude).

Thus, due to its piezoelectric nature, the AlGaN/GaN heterojunction structure can be successfully used as SAW sensors on the free standing AlGaN/GAN membrane. The use of the 2DEG/2DHG-based sensors within the SAW configuration enables a combination of ultrahigh sensitivity with excellent signal stability. It is well-known, that the SAW sensors are very sensitive to surface charges in the SAW propagation path between emitter and receiver finger-electrodes or IDTs. In addition, the SAW sensors have a very high Q-factor at the resonant frequency. The 2DEG/2DHG-based sensors increase an evanescent near-field acoustoelectric effect through the 2DEG/2DHG-density charge-responsivity following by drastic increase of sensitivity to proximal electrical charges. Moreover, the SAW sensors can be easily powered by an RF field with the corresponding frequency having an appropriate meander-based antenna. The SAW sensor offers the intrinsic RFID integration by using the orthogonal frequency coding.

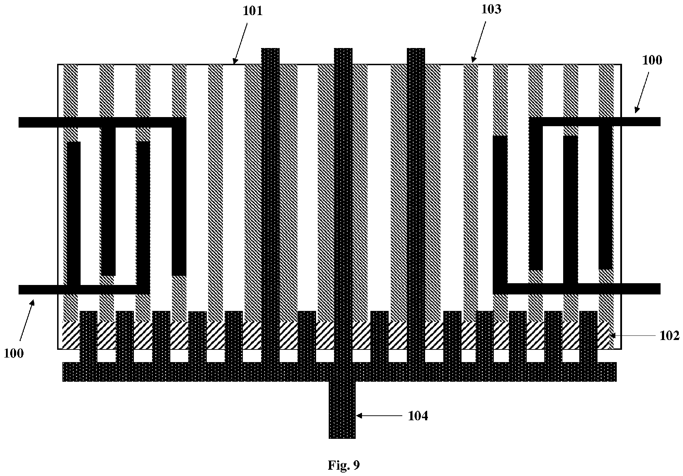

There is a large functional diversity of the 2DEG/2DHG-SAW RFID sensor topologies and layouts. The aim of the 2DEG/2DHG-SAW sensor topology is to achieve a largest influence of the SAW-transducer S21-transfer parameter without sacrificing the sensor stability. The basic topology of the sensor of the present invention is schematically shown in **Fig. 9****.** Dark bands (100) and (104) in **Fig. 9** are assigned to metal structures, such as metal IDTs (100) and metal electrodes (104), respectively, light striped bands (103) represent regular 2DEG/2DHG structures (103) (normally-on or normally-off HEMT-like structures), which are not pseudo-conducting (according to the definition), large striped bands (102) show pseudo-conducting 2DEG/2DHG structures (or PC-HEMT-like structures) (102), and white (background) area stands for the AlGaN/GaN-substrate (101).

Thus, in one aspect, the SAW RFID sensor chip of the present application comprises:
a piezoelectric substrate (101), said substrate comprising a piezoelectric layer and a multilayer heterojunction structure, said structure being made of III-V single-crystalline or polycrystalline semiconductor materials, deposited on said piezoelectric layer and comprising at least one buffer layer and at least one barrier layer, said layers being stacked alternately;
at least one pair of metal interdigitated transducers (IDT) (100) mounted on said piezoelectric substrate (101), for receiving a radio frequency (RF) input signal, transducing said input signal into a surface acoustic wave (SAW), propagating said surface acoustic wave along a surface of said piezoelectric substrate (101) and transducing said propagated surface acoustic wave into an output RF signal;
at least one normally-on or normally-off HEMT-like structure (103) deposited on said piezoelectric substrate (101) for forming a normally-on or normally-off 2DEG or 2DHG conducting channel in said heterojunction structure at the interface between said buffer layer and said barrier layer;
at least one PC-HEMT-like structure (102) deposited on said piezoelectric substrate (101) for forming the pseudo-conducting 2DEG or 2DHG channel in said heterojunction structure at the interface between said buffer layer and said barrier layer; and
electrical metallizations (not shown in the figure) capacitively-coupled to said IDTs (100) and to said HEMT-like structures (103) and/or PC-HEMT-like structures (102) for inducing displacement currents, thereby creating non-ohmic source and drain contacts, for connecting said sensor chip to an electric circuit.

The black IDT structures (100) receive the RF signal of about 0.5-2.5 GHz and exhibit the piezoelectric effect creating acoustic waves over the surface of the resonator. These surface acoustic waves propagate along the substrate with constructive interference from both input and output IDTs. The regular 2DEG/2DHG structures (103) are placed and patterned (connected) in such a manner as to electrically shortcut the positive and negative electric charges from running the SAW and to thereby considerably change or minimize the amplitude of the signal received on both IDTs via the direct piezoelectric effect. A metal gate electrode (104), which is connected to a body single point, is placed in such a manner as to exhibit the gating effect on the 2DEG/2DHG conducting channel of the AlGaN/GaN structures. To increase the gating effect, the areas under the metallic gate are recessed to pseudo-conducting state. By gating such 2DEG/2DHG structure, the electrical connection is interrupted via the body negative charges (mainly negative via the natural electrostatics). If the electrical connection within the 2DEG/2DHG structures is interrupted, then the parasitic effect of shortcutting SAW charges is no longer present, that changes the S21 transfer parameter, which is measured as the amplitude change on the receiver antenna device (could be a smart phone NFC chip).

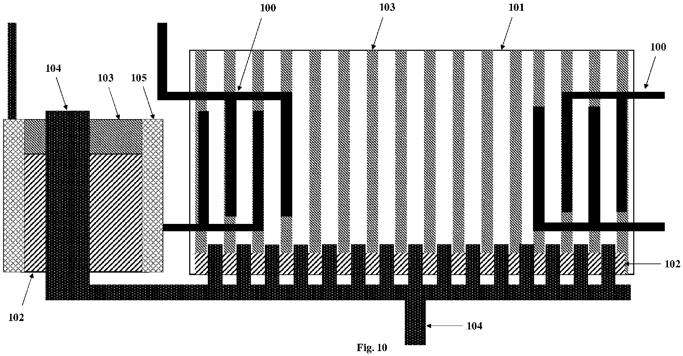

**Fig. 10** illustrates another basic topographic 2DEG/2DHG-SAW sensor chip configuration. The interconnection is integrated to supply the IDT (100) with the radio-frequency power. On the left, the PC-HEMT-like structure contains a major pseudo-conducting 2DEG/2DHG part (102) along with normal (non-recessed) 2DEG/2DHG part (103) to maintain a minimum SAW S21 stability. Capacitively-coupled contacts (105) are represented by the crossed-line bands. In this configuration, the maximal sensitivity is achieved with the maximal influence on the S21 transfer parameter by the single-point body charge. This is in turn achieved through the additional gating of the RF antenna power line for the IDT by means of the same gate electrode connected to the single body point.

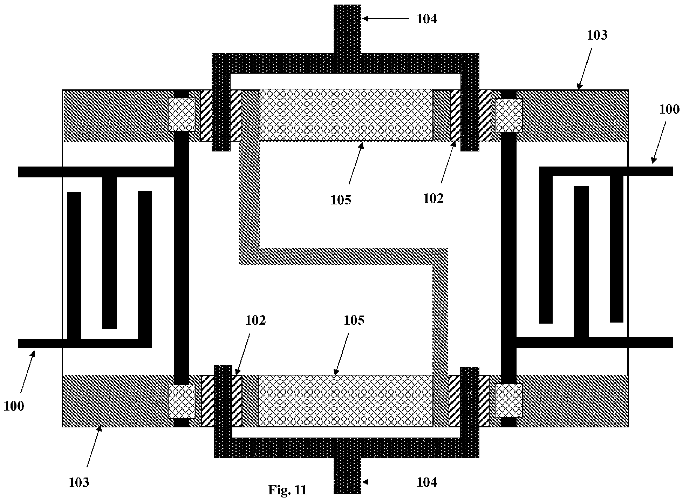

**Fig. 11** shows still another configuration of the 2DEG/2DHG-SAW sensor chip of an embodiment of the present application. In this configuration, the meander antenna parts for the signal and ground charge are separated by counter shortcutting each other via the parasitic 2DEG/2DHG lines (103) at the top and at the bottom of the shown layout. These lines are gated on the pseudo-conducting 2DEG/2DHG areas (102) by the gate electrode (104), which is in turn connected to a single body point (always negative). During this gating, the parasitic 2DEG/2DHG lines (103) are interrupted enabling the large increase of the S21 transfer parameter. The gating dynamics directly represents the final S21 amplitude dynamics and relates to the hemodynamic cardiovascular and pulmonary activity measured at the single body point.

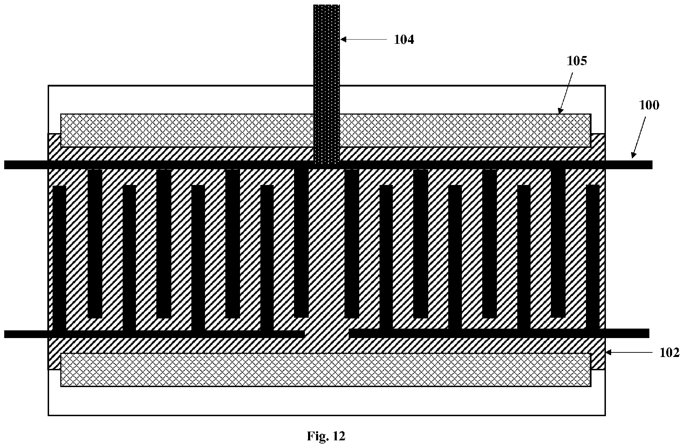

The last exemplary configuration of an embodiment is shown in **Fig. 12****.** In this simplest configuration, the pseudo-conducting 2DEG/2DHG area (102) is covering all the emitter-receiver IDTs area and is optionally connected via the non-ohmic contacts (105) to the gate electrode (104), which is in turn connected to the IDTs (100). The negative charge from a body will gate (deplete) the pseudo-conducting 2DEG/2DHG area (102), which is located beneath the Schottky metal surfaces, thereby minimising the parasitic shortcutting effect and tremendously increasing the influence on the S21 transfer parameter.

In all above configurations, the substrate (101) comprises a suitable material for forming the barrier layer and is composed, for example, of sapphire, silicon, silicon carbide, gallium nitride or aluminium nitride. The AlGaN/GaN heterojunction structure is deposited on this substrate layer, for example, by a method of metalorganic chemical vapour deposition (MOCVD). The non-recessed 2DEG/2DHG structures (103) are created in a close proximity to the interface between the GaN buffer layer and the AlGaN barrier layer. The specific thickness of the AlGaN barrier layer in the open gate area is achieved by either dry etching the semiconductor material of the layer, i.e. recessing layer in the open gate area with the etching rate of 1 nm per 1-2 min in a controllable process, or coating the AlGaN buffer layer with an ultrathin layer of the AlGaN semiconductor material. In order to increase the charge sensitivity of the sensor, the surface of the recessed ultrathin AlGaN layer is post-treated with plasma (chloride) epi-etch process. Consequently, the natively passivated surface is activated by the plasma etch to create an uncompensated (i.e. ionised) surface energy bonds or states, which are neutralized after the MOCVD growing.

The barrier layer then may be either recessed or grown as a thin layer to get the recessed 2DEG/2DHG structure (102). For example, the 2DEG channel formed at the interface between the buffer GaN layer and the barrier AlGaN layer serves as a main sensitive element of the sensor reacting to a surface charge and potential in the open gate area. The 2DEG channel is configured to interact with very small variations in surface or proximal charge or changes of electrical field as a result of the SAW creating a piezoelectric effect, and thereby, interacting with the donor-like surface trap states of the AlGaN barrier layer.

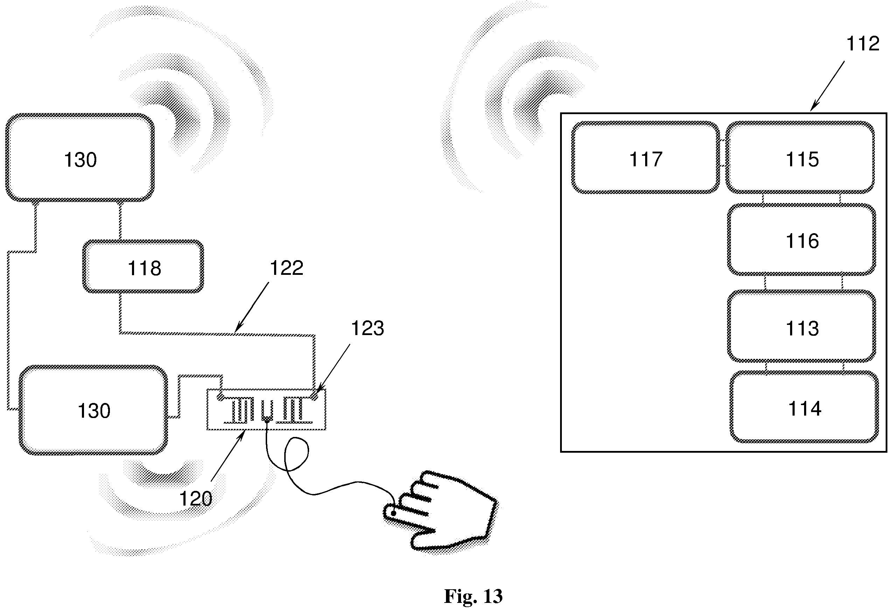

**Fig. 13** schematically shows a wearable device or gadget of the invention based on the zero-power SAW RFID sensor chip for remote readout, comprising the following components:
▪ the SAW sensor chip (120) of the application, inserted in a wearable (gadget) frame and connected via contacts (123) to an electric circuit (122);
▪ one or two out-input SAW-RFID zero-power fractal antennas (130), each connected to said contacts (123) via the electric circuit (122) for receiving or transmitting a signal;
▪ an output-input separation by delay line SAW transducer (118);
▪ an integrated circuit (112) for storing and processing said signal, and for modulating and demodulating a radio-frequency (RF) signals, said circuit comprising:
   a) a voltage source (114) supplying electric current to said SAW sensor chip (120) and to said one or two antennas (130);
   b) an integrated or CMOS current amplifier (115) for amplification of an electric current obtained from said SAW sensor chip (120);
   c) an analogue-to-digital converter (ADC) with wireless input/output modules (116) connected to said current amplifier (115) for wireless outputting the converted signal to a user interface or external memory;
   d) a microcontroller unit (MCU) (113) for processing and converting the received signal into data readable in said user interface or external memory; and
   e) a wireless connection module (117) for wireless connection of said sensor to said user interface or external memory.

The voltage source (114) can be any suitable and commercially available battery of the Li-ion type or any energy harvester with AC-DC or DC-DC converters. The ADC card (116) is any suitable analogue-to-digital converter data logger card that can be purchased, for example, from National Instruments® or LabJack®. The current amplifier (115) is connected in-line and can be any commercially available femtoampere amplifier, for example SRS® SR570, DLPVA-100-F-S, FEMTO® current amplifier DDPCA-300 or Texas Instruments® INA826EVM. Optionally, a current amplifier can be operated directly with current flowing via the 2DEG channel of the 2DEG structures into the amplifier with small input resistance of 1MΩ at gain higher than 10⁴ and only 1Ω at gains lower than 200. This setup may directly amplify the electric current modulation in the 2DEG channel originated from an external body charges. All readout components are battery powered to avoid ground loop parasitic current.

In a specific embodiment, the wireless connection module (117) can be a short-range Bluetooth or NFC providing wireless communication between the wearable device or gadget and a smartphone for up to 20 m. If this module is WiFi, the connection can be established with a network for up to 200 nm, while GSM allows the worldwide communication to a hemodynamic monitoring cloud or medical-diagnostic telemedicine cloud. The external memory may be a mobile device (such as a smartphone), desktop computer, server, remote storage, internet storage, hemodynamic monitoring cloud or medical-diagnostic telemedicine cloud.

In a further aspect of the present application, the sensor chip can be inserted in a wearable object or gadget, such as a bracelet, a ring, a neckband, a necklace, a pedant, an armband, a wristband or a clip on earring, applied to any available sensing point on user's body (arms, forearms, wrists, palms, fingers, earlobes, chest or neck). In a particular embodiment, the sensor of the application can be used for hemodynamic monitoring from any single point on a user's body and specifically from the wrist.

As shown in the present application, some embodiments of the sensors of the present application can be used in the hemodynamic monitoring, i.e. detecting, measuring and monitoring the cardiac signals and central venous pressure. Some embodiments of the sensors of the present application are also capable of recording a phonocardiogram. They are also capable of breath monitoring and lung activity diagnostics and hence, can be used in pulmonary and respiratory related applications.

In some embodiments, the wearable device of and the system of the present application can be used for portable long-time-operation solution within a health, fitness and remote telemedicine cloud-based diagnostics. Since the device is used in a hemodynamic monitoring, it should have a very small power consumption saving the battery life for a prolong usage. In this case, the ohmic contacts of the sensor chip can be replaced with non-ohmic high-resistive contacts capacitively connecting the chip to an electric circuit. The non-ohmic contacts actually limit an electric current flowing through the 2DEG/2DHG channel by having an electrical resistance 3-4 times higher than the resistance of the 2DEG/2DHG-channel, thereby reducing electrical power consumption without sacrificing sensitivity and functionality of the sensor. Thus, the use of non-ohmic contacts in some embodiments of the sensor of the present application is a hardware solution allowing to minimise the power consumption of the device. In another embodiment, the power consumption of the device can be minimised using a software algorithm managing the necessary recording time of the sensor and a battery saver mode, which limits the background data and switches the wireless connection only when it is needed.

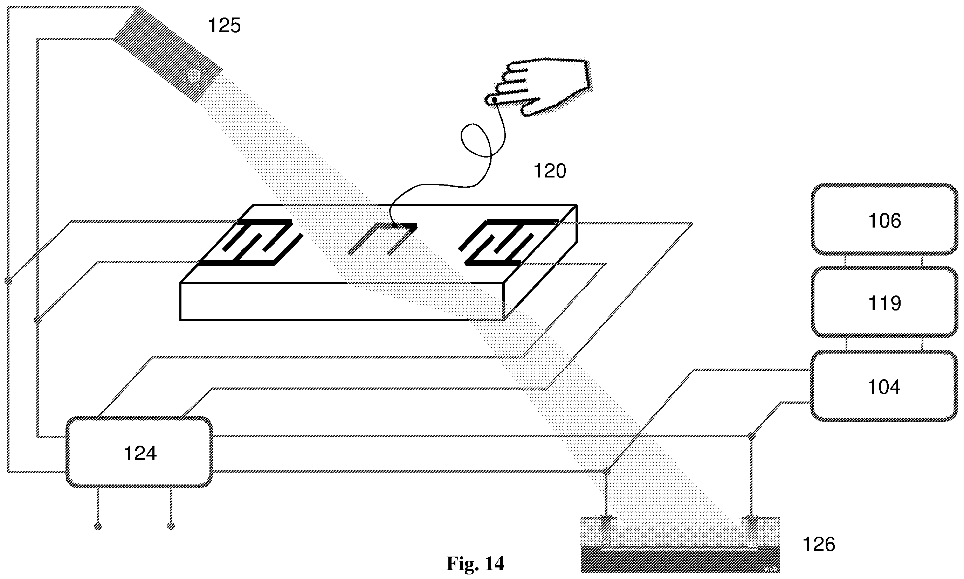

In an example, as depicted by **Fig. 14** schematically shows an optoelectronic sensor of the invention for remote readout comprising the following components:
▪ the SAW sensor chip (120) of the application connected to an electric circuit;
▪ a modulated light source (125), such as a surface-mounted-device light-emitting diode (SMD LED) or UV-VIS-IR laser diode, for irradiating the AlGaN barrier layer surface of the pseudo-conducting 2DEG structure (126) on the sensor chip;
▪ optocoupler switches (124) for coupling said modulated light source (125) with said pseudo-conducting 2DEG structure (126) on the sensor chip;
▪ a voltage source (104) connected to said electrical circuit for supplying electric current to said SAW sensor chip (120);
▪ a lock-in amplifier (119) connected to said voltage source (104) for amplification of a signal with a known carrier wave obtained from said SAW sensor chip and increasing the signal-to-noise ratio; and
▪ an analogue-to-digital converter (ADC) with in-built digital input/output card (106) connected to said lock-in amplifier (119) for outputting the converted signal to a user interface.

Alternatively, the SAW sensor may be based on a piezoelectric electro-optical crystal transducer (EOC) combined with the pseudo-conducting 2DEG-based structure for hemodynamic monitoring. The SAW device based on the EOC piezoelectric substrate exhibits the highest coupling between electrical and mechanical energy compared to all other varieties of substrates. Additionally, such a substrate also has the advantages of having a high velocity-shift coefficient and a very high electromechanical coupling coefficient, K2, which yields a greater mass sensitivity in comparison with the same regular SAW device on any other piezoelectric substrates. The EOC may be any suitable electro-optical crystalline material such as LiNbO₃, which is brought into a physical contact with a single point on a user's body. The EOC is then illuminated with a polarised light. In case of the LiNbO₃ crystalline material, the wavelength of the polarised light is about 400-600 nm. Modulated light from the light source illuminates the EOC, and then falls on the 2DEG/2DHG-based structure. The 2DEG/2DHG-based structure is extremely sensitive to an incident light creating the p-n-pairs in the AlGaN barrier layer and consequently, strongly affecting the 2DEG/2DHG-conductivity. In general, irradiation of the 2DEG/2DHG-based structure with light switches the 2DEG/2DHG-channel from normally-off to a pseudo-conducting or normally-on state. Therefore, by contact with a body, the EOC is capable of changing its light absorbance strongly affecting the electrical current flow in the 2DEG/2DHG channel, thereby resolving any smallest light intensity changes coming from the EOC transducer.

Due to the fact that hemodynamic signals are relatively slow and take time to be registered, the SAW sensor of an embodiment is able to track all the hemodynamics. By means of using the EOC-based configuration it is possible to fully decouple the conducting 2DEG/2DHG structure from any parasitic electrical charge coming from the human body. Depending on the excitation light wavelength, the position of the sensor relative to the incident light beam can be changed. For instance, in case of IR light (700-1500 nm), the sensor should be placed perpendicularly to the light beam for achieving the highest sensitivity. The parasitic charging of the EOC is compensated via the electrodes attached to the crystal. Additionally a variety of light filters in front of the sensor can be utilised.

Thus, the use of the SAW-EOC configuration makes it possible to drastically increase the sensitivity of the sensor to an electrical charge, to discharge the EOC via the SAW-based charge transport along the crystal surface, to efficiently modulate polarised light from the light source and to control the SAW delay line effect with the phase velocity signal. The opto-coupler switches (124) couples the pseudo-conducting 2DEG-based structure (126) with the SAW-EOC such that the initial SAW actuation signals at the emitter (left) IDT electrodes are synchronised with the modulated light source (125) and with the V_{DS} at the pseudo-conducting 2DEG/2DHG-based structure. A signal at the receiver (right) IDT electrodes is coupled back to the V_{DS} via the opto-coupler (124), which is brought into a resonance with initial signals and with the light source (125) modulation. Due to a physical galvanic connection of the SAW-EOC with the body single point by spatially patterned electrodes, the EOC changes its light absorption and modulation properties. This strongly affects the resonant mode of the five initial signal sources (V_{DS}, emitter IDT, light source, receiver IDT and SAW-modulated light source). Thus, because of the light source-based interaction, the resonant system becomes very stable and also very sensitive to external charges.

According to an example, a method for hemodynamic monitoring of a user comprises the following steps:
1) Applying the wearable device of the embodiments to a user's body;
2) Recording signals received from the user's body in a form of a S21-transfer parameter dynamics of the device over time (defined as S21-transfer dynamics) with said device;
3) Transmitting the recorded signals from said device to the external memory for further processing; and
4) Converting the transmitted signals to digital signals and processing the digital signals in the external memory, correlating said S21-transfer dynamics with pre-calibrated electrocardiogram and central venous pressure waveforms stored in the external memory, and extracting the user's cardiac signals and central venous pressure from said waveforms in a form of readable medical data, thereby providing hemodynamic medical information.

The S21-transfer dynamics may be further correlated with phonocardiogram waveforms stored in the external memory, thereby providing additional hemodynamic information on breath and lung activity relating to pulmonary and respiratory systems.

### REFERENCES

S. Nakayama, K. Sawamura, K. Mohri, T. Uchiyama, "Pulse-Driven Magnetoimpedance Sensor Detection of Cardiac Magnetic Activity", Plos One, Volume 6, Issue 10, e25834, 2011.
J. An, H. Li, L. Miao, S. Qin, "A Study on Human Magnetocardiogram Using Giant Magneto-impedance Sensor", Second International Conference on Electronics, Communications and Control, 2012.
Y. Kado, M. Shinagawa, "RedTacton Near-body Electric-field Communications Technology and Its Applications", NTT Technical Review, Vol. 8, No. 3, Mar. 2010.
S. D. Burnham, K. Boutros, P. Hashimoto, C. Butler, D. W. S. Wong, M. Hu, and M. Micovic, "Gate-recessed normally-off GaN-on-Si HEMT using a new O2-BCl3 digital etching technique", Phys. Status Solidi C, vol. 7, no. 7-8, pp. 2010-2012, 2010.
C. Y. Chang, S. J. Pearton, C. F. Lo, F. Ren, I. I. Kravchenko, A. M. Dabiran, A. M. Wowchak, B. Cui, and P. P. Chow, "Development of enhancement mode AlN/GaN high electron mobility transistors", Appl. Phys. Lett., vol. 94, no. 26, p. 263505, 2009.
H. Chen, M. Wang, and K. J. Chen, "Self-aligned enhancement-mode AlGaN/GaN HEMTs using 25 keV fluorine ion implantation", in Device Research Conference (DRC), 2010, pp. 137-138.
S. J. Pearton, B. S. Kang, Suku Kim, F. Ren, B. P. Gila, C. R. Abernathy, Jenshan Lin and S. N. G. Chu, "GaN-based diodes and transistors for chemical, gas, biological and pressure sensing", Journal of Physics: Condensed Matter, vol. 16 (2004), R961-R994.
N. Shigekawa, K. Nishimura, H. Yokoyama and K. Hohkawa, "Surface Acoustic Waves in Reverse-Biased AlGaN/GaN Heterostructures", IEEE Transactions - Electron Devices, vol. 55, no. 7, July 2008, pp. 1585-1591.

## Claims

1. A surface acoustic wave (SAW) radio-frequency identification (RFID) sensor chip comprising:
(a) a piezoelectric substrate (101), said substrate comprising a piezoelectric layer and a multilayer heterojunction structure, said structure being made of III-V single-crystalline or polycrystalline semiconductor layers, deposited on said piezoelectric layer and comprising at least one buffer layer and at least one barrier layer, said layers being stacked alternately;
(b) at least one pair of metal interdigitated transducers (IDT) (100) mounted on said piezoelectric substrate (101), for receiving a radio frequency (RF) input signal, transducing said input signal into a surface acoustic wave (SAW), propagating said surface acoustic wave along a surface of said piezoelectric substrate (101) and transducing said propagated surface acoustic wave into an output RF signal;
(c) at least one normally-on or normally-off two-dimensional electron gas (2DEG) or two-dimensional hole gas (2DHG) structure (103) deposited on said piezoelectric substrate (101) for forming a normally-on or normally-off 2DEG or 2DHG conducting channel in said multilayer heterojunction structure at the interface between said buffer layer and said barrier layer;
(d) at least one pseudo-conducting two-dimensional electron gas (2DEG) or two-dimensional hole gas (2DHG) structure (102) deposited on said piezoelectric substrate (101) for forming a pseudo-conducting 2DEG or 2DHG channel in said multilayer heterojunction structure at the interface between said buffer layer and said barrier layer; and
(e) electrical metallizations capacitively-coupled to said IDTs (100), to said normally-on or normally-off 2DEG or 2DHG structures (103) and to said pseudo-conducting 2DEG or 2DHG structures (102) for inducing displacement currents, thereby creating non-ohmic source and drain contacts, for connecting said sensor chip to an electric circuit;
***characterised in that:***
(i) said III-V single- or polycrystalline semiconductor layers are GaN/AlGaN; and
(ii) said pseudo-conducting 2DEG or 2DHG structure (102) is a semiconducting structure formed on said piezoelectric substrate (101) by recessing or growing a top layer of said multilayer heterojunction structure to a thickness of 5-9 nm with a surface roughness of the recessed or grown top layer being 0.2 nm or less, thereby forming the pseudo-conducting 2DEG or 2DHG channel capable of conducting electric current in the current range between normally-on and normally-off operation modes of the channel.

2. The SAW RFID sensor chip of claim 1, wherein said piezoelectric layer is made of zinc oxide, sapphire, aluminium nitride, lithium tantalate, lithium niobate, potassium niobate, lanthanum gallium silicate, silica, silicon carbide or quartz.

3. The SAW RFID sensor chip of claim 1, wherein said multilayer heterojunction structure contains one GaN buffer layer at the bottom and one AlGaN barrier layer at the top, said AlGaN barrier layer having (i) thickness of 5-9 nanometres (nm), corresponding to the pseudo-conducting current range between the normally-on and normally-off operation mode of the formed 2DEG channel, and (ii) surface roughness of 0.2 nm or less.

4. The SAW RFID sensor chip of claim 1, wherein said multilayer heterojunction structure is sandwich-like containing one GaN buffer layer at the top, one GaN buffer layer at the bottom and one AlGaN barrier layer in between, said 2DEG conducting channel being formed in the top GaN buffer layer above the AlGaN barrier layer, close to the interface between said top GaN buffer layer and said AlGaN barrier layer, thereby resulting in a N-face polarity of said structure, said top GaN buffer layer having (i) thickness of 5-9 nanometres (nm), corresponding to the pseudo-conducting current range between the normally-on and normally-off operation mode of the formed 2DEG channel, and (ii) surface roughness of 0.2 nm or less.

5. The SAW RFID sensor chip of claim 1, wherein said multilayer heterojunction structure is sandwich-like containing one GaN buffer layer at the top, one GaN buffer layer at the bottom and one AlGaN barrier layer in between, said 2DHG conducting channel being formed in the top GaN buffer layer above the AlGaN barrier layer, close to the interface between said top GaN buffer layer and said AlGaN barrier layer, thereby resulting in a Ga-face polarity of said structure, said top GaN buffer layer having (i) thickness of 5-9 nanometres (nm), which corresponds to the pseudo-conducting current range between the normally-on and normally-off operation mode of the formed 2DHG channel, and (ii) surface roughness of 0.2 nm or less.

6. The SAW RFID sensor chip of any one of claims 1-5, wherein the thickness of the top layer is 6-7 nm, preferably 6.2-6.4 nm.

7. The SAW RFID sensor chip of any one of claims 1-6, wherein said top layer has the surface roughness of about 0.1 nm or less, preferably about 0.05 nm or less.

8. The SAW RFID sensor chip of any one of claims 1-7, further comprising an excitation light source for irradiating said piezoelectric substrate, thereby inducing an electric current in said 2DEG or 2DHG structure.

9. The SAW RFID sensor chip of claim 8, wherein said excitation light source is a surface-mounted-device light-emitting diode (SMD LED) or UV-VIS-IR laser diode.

10. The SAW RFID sensor chip of any one of claims 1-9, wherein said metal IDTs (100) are capable of receiving the RF signal of about 0.5-2.5 GHz and exhibiting the piezoelectric effect by creating acoustic waves over the surface of said piezoelectric substrate.

11. A wearable device with a remote readout, comprising:
▪ the SAW RFID sensor chip (120) of any one of claims 1-10, inserted in a wearable device frame and connected to an electric circuit (122);
▪ at least one out-input SAW-RFID zero-power fractal antenna (130) connected to said electric circuit (122), for receiving or transmitting a signal;
▪ an output-input separation by delay line SAW transducer (118);
▪ a remote integrated circuit (112) for storing and processing said signal, and for modulating and demodulating a radio-frequency (RF) signals, said remote integrated circuit comprising:
a) a voltage source (114) supplying electric current to said SAW RFID sensor chip (120) and to said out-input SAW-RFID zero-power fractal antenna/s (130);
b) an integrated or CMOS current amplifier (115) for amplification of an electric current obtained from said SAW RFID sensor chip (120);
c) an analogue-to-digital converter with wireless input/output modules (116) connected to said current amplifier (115) for wireless outputting the converted signal to a user interface or external memory;
d) a microcontroller unit (MCU) (113) for processing and converting the received signal into data readable in said user interface or external memory; and
e) a wireless connection module (117) for wireless connection of said wearable device to said user interface or external memory.

12. The wearable device of claim 11, wherein said wireless connection module (117) is a short-range Bluetooth® or NFC module providing wireless communication between said sensing device and the user interface, mobile device or desktop computer; or a Wi-Fi module providing wireless communication between said sensing device and the user interface, a mobile device, desktop computer or server; or a GSM module providing a worldwide wireless communication between said sensing device and a server, remote storage, internet storage, hemodynamic monitoring cloud or medical-diagnostic telemedicine cloud.

13. The wearable device of claim 11, wherein said wearable device is in a form of a bracelet, a ring, a neckband, a necklace, a pedant, an armband, a wristband or a clip on earring.

14. A method for hemodynamic monitoring of a user comprising:
1) Applying the wearable device of any one of claims 11-13 to the user's body, arm, forearm, wrist, palm, finger, earlobe, chest or neck;
2) Recording signals received from the user's body in a form of a S21-transfer parameter dynamics of the device over time with said device;
3) Transmitting the recorded signals from said device to the external memory for further processing; and
4) Converting the transmitted signals to digital signals and processing the digital signals in the external memory, correlating said S21-transfer dynamics with pre-calibrated electrocardiogram and central venous pressure waveforms stored in the external memory, and extracting the user's cardiac signals and central venous pressure from said waveforms in a form of readable medical data, thereby providing hemodynamic medical information.

15. The method of claim 14, wherein the S21-transfer dynamics is further correlated with phonocardiogram waveforms stored in the external memory, thereby providing additional hemodynamic information on breath and lung activity relating to pulmonary and respiratory systems.

## Patentansprüche

1. Sensorchip zur Radiofrequenzidentifikation (RFID) mittels akustischer Oberflächenwellen (SAW), umfassend:
(a) ein piezoelektrisches Substrat (101), wobei das Substrat eine piezoelektrische Schicht und eine mehrschichtige Heteroübergangsstruktur umfasst, wobei die Struktur aus monokristallinen oder polykristallinen III-V-Halbleiterschichten hergestellt ist, die auf die piezoelektrische Schicht abgeschieden sind und mindestens eine Pufferschicht und mindestens eine Sperrschicht umfassen, wobei die Schichten abwechselnd gestapelt sind;
(b) mindestens ein Paar von Metall-Interdigitalwandlern (IDT) (100), das auf dem piezoelektrischen Substrat (101) montiert ist, zum Empfangen eines Radiofrequenz (RF)-Eingangssignals, Umwandeln des Eingangssignals in eine akustische Oberflächenwelle (SAW), Propagieren der akustischen Oberflächenwelle entlang einer Oberfläche des piezoelektrischen Substrats (101) und Umwandeln der propagierten akustischen Oberflächenwelle in ein RF-Ausgangssignal;
(c) mindestens eine selbstleitende oder selbstsperrende zweidimensionale Elektronengas (2DEG)- oder zweidimensionale Lochgas (2DHG)-Struktur (103), die auf das piezoelektrische Substrat (101) abgeschieden ist, zum Bilden eines selbstleitenden oder selbstsperrenden 2DEG- oder 2DHG-Leitungskanals in der mehrschichtigen Heteroübergangsstruktur an der Grenzfläche zwischen der Pufferschicht und der Sperrschicht;
(d) mindestens eine pseudoleitende zweidimensionale Elektronengas (2DEG)- oder zweidimensionale Lochgas (2DHG)-Struktur (102), die auf das piezoelektrische Substrat (101) abgeschieden ist, zum Bilden eines pseudoleitenden 2DEG- oder 2DHG-Kanals in der mehrschichtigen Heteroübergangsstruktur an der Grenzfläche zwischen der Pufferschicht und der Sperrschicht; und
(e) elektrische Metallisierungen, die mit den IDTs (100), mit den selbstleitenden oder selbstsperrenden 2DEG- oder 2DHG-Strukturen (103) und mit den pseudoleitenden 2DEG- oder 2DHG-Strukturen (102) kapazitiv gekoppelt sind, zum Induzieren von Verschiebungsströmen, um dadurch nicht-ohmsche Source- und Drain-Kontakte zum Verbinden des Sensorchips mit einer elektrischen Schaltung zu bilden;
**dadurch gekennzeichnet, dass**:
(i) es sich bei den mono- oder polykristallinen III-V-Halbleiterschichten um GaN/AlGaN handelt; und
(ii) die pseudoleitende 2DEG- oder 2DHG-Struktur (102) eine Halbleiterstruktur ist, die auf dem piezoelektrischen Substrat (101) durch Versenken oder Aufwachsen einer obersten Schicht der mehrschichtigen Heteroübergangsstruktur bis zu einer Dicke von 5 bis 9 nm gebildet ist, wobei eine Oberflächenrauheit der versenkten oder aufgewachsenen Obersten Schicht 0,2 nm oder weniger beträgt, um dadurch den pseudoleitenden 2DEG- oder 2DHG-Kanal zu bilden, der zum Leiten von elektrischem Strom im Strombereich zwischen selbstleitenden und selbstsperrenden Betriebsmodi des Kanals imstande ist.

2. SAW-RFID-Sensorchip nach Anspruch 1, wobei die piezoelektrische Schicht aus Zinkoxid, Saphir, Aluminiumnitrid, Lithiumtantalat, Lithiumniobat, Kaliumniobat, Lanthanumgalliumsilicat, Silica, Siliciumcarbid oder Quarz hergestellt ist.

3. SAW-RFID-Sensorchip nach Anspruch 1, wobei die mehrschichtige Heteroübergangsstruktur eine GaN-Pufferschicht auf der Unterseite und eine AIGaN-Sperrschicht auf der Oberseite enthält, wobei die AlGaN-Sperrschicht (i) eine Dicke von 5 bis 9 Nanometer (nm), die dem pseudoleitenden Strombereich zwischen dem selbstleitenden und dem selbstsperrenden Betriebsmodus des gebildeten 2DEG-Kanals entspricht, und (ii) eine Oberflächenrauheit von 0,2 nm oder weniger aufweist.

4. SAW-RFID-Sensorchip nach Anspruch 1, wobei die mehrschichtige Heteroübergangsstruktur sandwichartig eine GaN-Pufferschicht auf der Oberseite, eine GaN-Pufferschicht auf der Unterseite und eine AlGaN-Sperrschicht dazwischen enthält, wobei der 2DEG-Leitungskanal in der oberen GaN-Pufferschicht über der AIGaN-Sperrschicht in der Nähe der Grenzfläche zwischen der oberen GaN-Pufferschicht und der AlGaN-Sperrschicht ausgebildet ist, woraus eine N-face-Polarität der Struktur resultiert, wobei die obere GaN-Pufferschicht (i) eine Dicke von 5 bis 9 Nanometer (nm), die dem pseudoleitenden Strombereich zwischen dem selbstleitenden und dem selbstsperrenden Betriebsmodus des gebildeten 2DEG-Kanals entspricht, und (ii) eine Oberflächenrauheit von 0,2 nm oder weniger aufweist.

5. SAW-RFID-Sensorchip nach Anspruch 1, wobei die mehrschichtige Heteroübergangsstruktur sandwichartig eine GaN-Pufferschicht auf der Oberseite, eine GaN-Pufferschicht auf der Unterseite und eine AlGaN-Sperrschicht dazwischen enthält, wobei der 2DHG-Leitungskanal in der oberen GaN-Pufferschicht über der AIGaN-Sperrschicht in der Nähe der Grenzfläche zwischen der oberen GaN-Pufferschicht und der AlGaN-Sperrschicht ausgebildet ist, woraus eine Ga-face-Polarität der Struktur resultiert, wobei die obere GaN-Pufferschicht (i) eine Dicke von 5 bis 9 Nanometer (nm), die dem pseudoleitenden Strombereich zwischen dem selbstleitenden und dem selbstsperrenden Betriebsmodus des gebildeten 2DHG-Kanals entspricht, und (ii) eine Oberflächenrauheit von 0,2 nm oder weniger aufweist.

6. SAW-RFID-Sensorchip nach einem der Ansprüche 1 bis 5, wobei die Dicke der obersten Schicht 6 bis 7 nm, vorzugsweise 6,2 bis 6,4 nm beträgt.

7. SAW-RFID-Sensorchip nach einem der Ansprüche 1 bis 6, wobei die oberste Schicht eine Oberflächenrauheit von etwa 0,1 nm oder weniger, vorzugsweise etwa 0,05 nm oder weniger aufweist.

8. SAW-RFID-Sensorchip nach einem der Ansprüche 1 bis 7, ferner umfassend eine Anregungslichtquelle zum Bestrahlen des piezoelektrischen Substrats, um dadurch einen elektrischen Strom in der 2DEG- oder der 2DHG-Struktur zu induzieren.

9. SAW-RFID-Sensorchip nach Anspruch 8, wobei die Anregungslichtquelle ein oberflächenmontiertes Leuchtdioden-Bauelement (SMD-LED) oder eine UV-VIS-IR-Laserdiode ist.

10. SAW-RFID-Sensorchip nach einem der Ansprüche 1 bis 7, wobei die Metall-IDTs (100) zum Empfangen des RF-Signals von etwa 0,5 bis 2,5 GHz imstande sind und den piezoelektrischen Effekt durch Erzeugen von akustischen Wellen über der Oberfläche des piezoelektrischen Substrats aufweisen.

11. Am Körper tragbare Vorrichtung mit Fernauslesung, umfassend:
▪ den SAW-RFID-Sensorchip (120) nach einem der Ansprüche 1 bis 10, der in einen Rahmen einer am Körper tragbaren Vorrichtung eingefügt und mit einer elektrischen Schaltung (122) verbunden ist;
▪ mindestens eine Ein-/Ausgangs-SAW-RFID-Nullleistungs-Fraktalantenne (130), die mit der elektrischen Schaltung (122) verbunden ist, zum Empfangen oder Senden eines Signals;
▪ eine Ein-/Ausgangs-Trennung durch einen SAW-Wandler (118) einer Verzögerungsleitung;
▪ eine integrierte Remoteschaltung (112) zum Speichern und Verarbeiten des Signals und zum Modulieren und Demodulieren eines Radiofrequenz (RF)-Signals, wobei die integrierte Remoteschaltung umfasst:
a) eine Spannungsquelle (114), die den SAW-RFID-Sensorchip (120) und die Ein-/Ausgangs-SAW-RFID-Nullleistungs-Fraktalantennen (130) mit elektrischem Strom versorgt;
b) einen integrierten oder CMOS-Stromverstärker (115), der mit der Spannungsquelle (104) verbunden ist, zur Verstärkung eines elektrischen Stroms, der vom SAW-RFID-Sensorchip (120) erhalten wird;
c) einen Analog-Digital-Konverter mit drahtlosen Eingabe-/Ausgabe-Modulen (116), der mit dem Stromverstärker (115) verbunden ist, zum drahtlosen Ausgeben des konvertierten Signals an eine Benutzerschnittstelle oder einen externen Speicher;
d) eine Mikrocontroller-Einheit (MCU) (113) zum Verarbeiten und Konvertieren des empfangenen Signals in Daten, die in der Benutzerschnittstelle oder dem externen Speicher gelesen werden können; und
e) ein Drahtlosverbindungsmodul (117) zur drahtlosen Verbindung der am Körper tragbaren Vorrichtung mit der Benutzerschnittstelle oder dem externen Speicher.

12. Am Körper tragbare Vorrichtung nach Anspruch 11, wobei das Drahtlosverbindungsmodul (117) ein Nahbereichs-Bluetooth®- oder -NFC-Modul, das drahtlose Kommunikation zwischen der Sensorvorrichtung und der Benutzerschnittstelle, einer Mobilvorrichtung oder einem Desktop-Computer bereitstellt; oder ein Wi-Fi-Modul, das drahtlose Kommunikation zwischen der Sensorvorrichtung und der Benutzerschnittstelle, einer Mobilvorrichtung, einem Desktop-Computer oder einem Server bereitstellt; oder ein GSM-Modul ist, das weltweite drahtlose Kommunikation zwischen der Sensorvorrichtung und einem Server, einem Remotespeicher, einem Internetspeicher, einer Hämodynamik-Überwachungs-Cloud oder einer medizinischdiagnostischen Telemedizin-Cloud bereitstellt.

13. Am Körper tragbare Vorrichtung nach Anspruch 11, wobei die am Körper tragbare Vorrichtung in Form eines Armreifs, eines Rings, eines Halsbands, einer Halskette, eines Anhängers, eines Armbands, einer Manschette oder eines Clips auf einem Ohrring ist.

14. Verfahren zur hämodynamischen Überwachung eines Benutzers, umfassend:
1) Anbringen der am Körper tragbaren Vorrichtung nach einem der Ansprüche 11 bis 13 am Körper, einem Arm, einem Unterarm, einem Handgelenk, einer Handfläche, einem Finger, einem Ohrläppchen, der Brust oder dem Nacken des Benutzers;
2) Aufzeichnen von Signalen, die vom Körper des Benutzers in Form einer S21-Übertragungsparameterdynamik der Vorrichtung im Zeitablauf empfangen werden, mit der Vorrichtung;
3) Senden der aufgezeichneten Signale von der Vorrichtung an den externen Speicher zur Weiterverarbeitung; und
4) Konvertieren der gesendeten Signale in digitale Signale und Verarbeiten der digitalen Signale im externen Speicher, Korrelieren der S21-Übertragungsdynamik mit vorkalibrierten Elektrokardiogramm- und Zentralvenendruck-Wellenformen, die im externen Speicher gespeichert sind, und Extrahieren der Herzsignale und des zentralen Venendrucks aus den Wellenformen in Form von lesbaren medizinischen Daten, um dadurch hämodynamische medizinische Informationen bereitzustellen.

15. Verfahren nach Anspruch 14, wobei die S21-Übertragungsdynamik ferner mit Phonokardiogramm-Wellenformen korreliert wird, die im externen Speicher gespeichert sind, um dadurch zusätzliche hämodynamische Informationen über die Atem- und Lungenaktivität in Bezug auf das Lungen- und das Atmungssystem bereitzustellen.

## Revendications

1. Puce de capteur d'identification par radiofréquence (RFID) à ondes acoustiques de surface (SAW) comprenant :
(a) un substrat piézoélectrique (101), ledit substrat comprenant une couche piézoélectrique et une structure à hétérojonction multicouche, ladite structure étant constituée de couches semi-conductrices polycristallines ou monocristallines III-V, déposées sur ladite couche piézoélectrique, et comprenant au moins une couche tampon et au moins une couche barrière, lesdites couches étant empilées de manière alternée ;
(b) au moins une paire de transducteurs interdigités métalliques (IDT) (100) montés sur ledit substrat piézoélectrique (101), en vue de recevoir un signal d'entrée radiofréquence (RF), de convertir ledit signal d'entrée en une onde acoustique de surface (SAW), de propager ladite onde acoustique de surface le long d'une surface dudit substrat piézoélectrique (101) et de convertir ladite onde acoustique de surface propagée en un signal RF de sortie ;
(c) au moins une structure de gaz électronique bidimensionnel (2DEG) ou de gaz de trou bidimensionnel (2DHG) normalement active ou normalement inactive (103), déposée sur ledit substrat piézoélectrique (101) pour former un canal conducteur de gaz 2DEG ou 2DHG normalement actif ou normalement inactif dans ladite structure à hétérojonction multicouche au niveau de l'interface entre ladite couche tampon et ladite couche barrière ;
(d) au moins une structure de gaz électronique bidimensionnel (2DEG) ou de gaz de trou bidimensionnel (2DHG) pseudo-conductrice (102) déposée sur ledit substrat piézoélectrique (101) pour former un canal de gaz 2DEG ou 2DHG pseudo-conducteur dans ladite structure à hétérojonction multicouche au niveau de l'interface entre ladite couche tampon et ladite couche barrière ; et
(e) des métallisations électriques couplées de manière capacitive auxdits transducteurs IDT (100), auxdites structures de gaz 2DEG ou 2DHG normalement actives ou normalement inactives (103) et auxdites structures de gaz 2DEG ou 2DHG pseudo-conductrices (102) pour induire des courants de déplacement, ce qui permet de créer par conséquent des contacts de source et de drain non ohmiques, pour connecter ladite puce de capteur à un circuit électrique ;
***caractérisé en ce que* :**
(i) lesdites couches semi-conductrices polycristallines ou monocristallines III-V sont constituées de GaN/AlGaN ; et
(ii) ladite structure de gaz 2DEG ou 2DHG pseudo-conductrice (102) est une structure semi-conductrice formée sur ledit substrat piézoélectrique (101) par évidement ou croissance d'une couche supérieure de ladite structure à hétérojonction multicouche jusqu'à une épaisseur de 5-9 nm, où une rugosité de surface de la couche supérieure à évidement ou à croissance est égale à 0,2 nm ou moins, ce qui permet de former par conséquent le canal de gaz 2DEG ou 2DHG pseudo-conducteur en mesure de conduire un courant électrique dans la plage de courant entre les modes de fonctionnement normalement actif et normalement inactif du canal.

2. Puce de capteur d'identification RFID à ondes SAW selon la revendication 1, dans laquelle ladite couche piézoélectrique est constituée d'oxyde de zinc, de saphir, de nitrure d'aluminium, de tantalate de lithium, de niobate de lithium, de niobate de potassium, de silicate de lanthane-gallium, de silice, de carbure de silicium ou de quartz.

3. Puce de capteur d'identification RFID à ondes SAW selon la revendication 1, dans laquelle ladite structure à hétérojonction multicouche contient une couche tampon en GaN dans la partie inférieure et une couche barrière en AIGaN dans la partie supérieure, ladite couche barrière en AIGaN présentant (i) une épaisseur de 5-9 nanomètres (nm), ce qui correspond à la plage de courant pseudo-conducteur entre le mode de fonctionnement normalement actif et normalement inactif du canal de gaz 2DEG formé, et (ii) une rugosité de surface de 0,2 nm ou moins.

4. Puce de capteur d'identification RFID à ondes SAW selon la revendication 1, dans laquelle ladite structure à hétérojonction multicouche est de type « sandwich », et contient une couche tampon en GaN dans la partie supérieure, une couche tampon en GaN dans la partie inférieure, et une couche barrière en AIGaN entre elles, ledit canal conducteur de gaz 2DEG étant formé dans la couche tampon en GaN supérieure au-dessus de la couche barrière en AIGaN, à proximité de l'interface entre ladite couche tampon en GaN supérieure et ladite couche barrière en AIGaN, ce qui se traduit par conséquent par une polarité de face N de ladite structure, ladite couche tampon en GaN supérieure présentant (i) une épaisseur de 5-9 nanomètres (nm), correspondant à la plage de courant pseudo-conducteur entre le mode de fonctionnement normalement actif et normalement inactif du canal de gaz 2DEG formé, et (ii) une rugosité de surface de 0,2 nm ou moins.

5. Puce de capteur d'identification RFID à ondes SAW selon la revendication 1, dans laquelle ladite structure à hétérojonction multicouche est de type « sandwich » et contient une couche tampon en GaN dans la partie supérieure, une couche tampon en GaN dans la partie inférieure, et une couche barrière en AIGaN entre elles, ledit canal conducteur de gaz 2DHG étant formé dans la couche tampon en GaN supérieure au-dessus de la couche barrière en AIGaN, à proximité de l'interface entre ladite couche tampon en GaN supérieure et ladite couche barrière en AIGaN, ce qui se traduit par conséquent par une polarité de face Ga de ladite structure, ladite couche tampon en GaN supérieure présentant (i) une épaisseur de 5-9 nanomètres (nm), ce qui correspond à la plage de courant pseudo-conducteur entre le mode de fonctionnement normalement actif et normalement inactif du canal de gaz 2DHG formé, et (ii) une rugosité de surface de 0,2 nm ou moins.

6. Puce de capteur d'identification RFID à ondes SAW selon l'une quelconque des revendications 1 à 5, dans laquelle l'épaisseur de la couche supérieure est de 6-7 nm, de préférence de 6,2-6,4 nm.

7. Puce de capteur d'identification RFID à ondes SAW selon l'une quelconque des revendications 1 à 6, dans laquelle ladite couche supérieure présente une rugosité de surface d'environ 0,1 nm ou moins, de préférence d'environ 0,05 nm ou moins.

8. Puce de capteur d'identification RFID à ondes SAW selon l'une quelconque des revendications 1 à 7, comprenant en outre une source de lumière d'excitation pour irradier ledit substrat piézoélectrique, ce qui induit par conséquent un courant électrique dans ladite structure de gaz 2DEG ou 2DHG.

9. Puce de capteur d'identification RFID à ondes SAW selon la revendication 8, dans laquelle ladite source de lumière d'excitation est une diode électroluminescente de dispositif monté en surface (SMD LED) ou une diode laser UV-VIS-IR.

10. Puce de capteur d'identification RFID à ondes SAW selon l'une quelconque des revendications 1 à 9, dans laquelle lesdits transducteurs IDT métalliques (100) sont en mesure de recevoir le signal RF d'environ 0,5-2,5 GHz et de présenter l'effet piézoélectrique en créant des ondes acoustiques sur la surface dudit substrat piézoélectrique.

11. Dispositif vestimentaire doté d'un affichage à distance, comprenant :
▪ la puce de capteur d'identification RFID à ondes SAW (120) selon l'une quelconque des revendications 1 à 10, insérée dans un cadre de dispositif vestimentaire et connectée à un circuit électrique (122) ;
▪ au moins une antenne fractale à puissance nulle d'identification RFID à ondes SAW d'entrée-sortie (130) connectée audit circuit électrique (122), pour recevoir ou transmettre un signal ;
× une séparation d'entrée-sortie par un transducteur SAW à ligne à retard (118) ;
▪ un circuit intégré distant (112) pour stocker et traiter ledit signal, et pour moduler et démoduler des signaux radiofréquence (RF), ledit circuit intégré distant comprenant :
a) une source de tension (114) fournissant un courant électrique à ladite puce de capteur d'identification RFID à ondes SAW (120) et à ladite ou auxdites antennes fractales à puissance nulle d'identification RFID à ondes SAW d'entrée-sortie (130) ;
b) un amplificateur de courant intégré ou CMOS (115) permettant l'amplification d'un courant électrique obtenu à partir de ladite puce de capteur d'identification RFID à ondes SAW (120) ;
c) un convertisseur analogique-numérique avec des modules d'entrée/sortie sans fil (116) connectés audit amplificateur de courant (115) en vue de fournir en sortie, par voie hertzienne, le signal converti, à une interface utilisateur ou à une mémoire externe ;
d) une unité de microcontrôleur (MCU) (113) pour traiter et convertir le signal reçu en des données lisibles dans ladite interface utilisateur ou ladite mémoire externe ; et
e) un module de connexion sans fil (117) pour la connexion sans fil dudit dispositif vestimentaire à ladite interface utilisateur ou à la mémoire externe.

12. Dispositif vestimentaire selon la revendication 11, dans lequel ledit module de connexion sans fil (117) est un module Bluetooth® ou NFC à courte portée fournissant une communication sans fil entre ledit dispositif de détection et l'interface utilisateur, un dispositif mobile ou un ordinateur de bureau ; ou un module Wi-Fi fournissant une communication sans fil entre ledit dispositif de détection et l'interface utilisateur, un dispositif mobile, un ordinateur de bureau ou un serveur ; ou un module GSM fournissant une communication sans fil mondiale entre ledit dispositif de détection et un serveur, un magasin de stockage à distance, un magasin de stockage sur Internet, un nuage informatique de surveillance hémodynamique ou un nuage informatique de télémédecine de diagnostic médical.

13. Dispositif vestimentaire selon la revendication 11, dans lequel ledit dispositif vestimentaire se présente sous la forme d'un bracelet, d'une bague, d'un collier, d'un pendentif, d'un brassard, d'un poignet ou d'une boucle d'oreille à clip.

14. Procédé de surveillance hémodynamique d'un utilisateur comprenant les étapes ci-dessous consistant à :
1) appliquer le dispositif vestimentaire selon l'une quelconque des revendications 11 à 13 sur le corps, le bras, l'avant-bras, le poignet, la paume, le doigt, le lobe de l'oreille, la poitrine ou le cou de l'utilisateur ;
2) enregistrer des signaux reçus en provenance du corps de l'utilisateur sous la forme d'une dynamique de paramètres de transfert S21 du dispositif au fil du temps, avec ledit dispositif ;
3) transmettre les signaux enregistrés, dudit dispositif à la mémoire externe, en vue d'un traitement ultérieur ; et
4) convertir les signaux transmis en des signaux numériques, et traiter les signaux numériques dans la mémoire externe, corréler ladite dynamique de paramètres de transfert S21 avec des formes d'onde de pression veineuse centrale et d'électrocardiogramme précalibré stockées dans la mémoire externe, et extraire les signaux cardiaques et la pression veineuse centrale de l'utilisateur, à partir desdites formes d'onde, sous la forme de données médicales lisibles, ce qui permet de fournir par conséquent des informations médicales hémodynamiques.

15. Procédé selon la revendication 14, dans lequel la dynamique de paramètres de transfert S21 est en outre corrélée avec des formes d'onde de phono-cardiogramme stockées dans la mémoire externe, ce qui permet de fournir par conséquent des informations hémodynamiques supplémentaires sur la respiration et l'activité pulmonaire connexes à des systèmes pulmonaire et respiratoire.
